# EUROPEAN PATENT SPECIFICATION

(11) **EP 2 902 498 B1**
(45) Date of publication and mention of the grant of the patent: **02.01.2019**
(21) Application number: 13795809.6
(22) Date of filing: 27.09.2013
(51) Int. Cl.: C12Q 1/6876

(54) **POLYMORPHISMS ASSOCIATED WITH THE DEGREE OF UNSATURATION OF INTRAMUSCULAR FAT IN PIGS**
POLYMORPHISMEN IN ZUSAMMENHANG MIT DEM GRAD DER UNGESÄTTIGTHEIT VON INTRAMUSKULÄREN FETT BEI SCHWEINEN
POLYMORPHISMES ASSOCIÉS AU DEGRÉ D'INSATURATION DE LA GRAISSE INTRAMUSCULAIRE CHEZ DES PORCS

(30) Priority: 28.09.2012 ES 201231507
(43) Date of publication of application: 05.08.2015
(73) Proprietor: Universitat de Lleida, 25003 Lleida (ES)
(72) Inventor: ESTANY ILLA, Joan, E-25003 LLeida (ES); PENA SUBIRÀ, Ramona Natacha, E-25003 LLeida (ES); TOR NAUDI, Marc, Lleida (ES)
(74) Representative: ABG Intellectual Property, S.L.
(86) International application number: PCT/ES2013/070670
(87) International publication number: WO 2014/049191

(56) References cited:
- EP-A1- 1 598 430
- KR-A- 20120 072 871
- REN J ET AL: "Isolation and molecular characterization of the porcine stearoyl-CoA desaturase gene", GENE, ELSEVIER, AMSTERDAM, NL, vol. 340, no. 1, 29 September 2004 (2004-09-29), pages 19-30, XP004649111, ISSN: 0378-1119, DOI: 10.1016/J.GENE.2004.06.017 -& Ren J ET AL: "Sus scrofa stearoyl-CoA desaturase (SCD) gene, exons 1 through 6 and complete cds - AY487830", Genbank, 15 September 2004 (2004-09-15), XP055095174, Retrieved from the Internet: URL:http://www.ncbi.nlm.nih.gov/nuccore/ay 487830 [retrieved on 2014-01-08]
- Y. UEMOTO ET AL: "Fine mapping of porcine SSC14 QTL and SCD gene effects on fatty acid composition and melting point of fat in a Duroc purebred population", ANIMAL GENETICS, vol. 43, no. 2, 1 August 2011 (2011-08-01) , pages 225-228, XP055095080, ISSN: 0268-9146, DOI: 10.1111/j.1365-2052.2011.02236.x cited in the application
- J. REN ET AL: "Characterization of five single nucleotide polymorphisms in the porcine stearoyl-CoA desaturase (SCD) gene", ANIMAL GENETICS, vol. 35, no. 3, 1 June 2004 (2004-06-01), pages 255-257, XP055095097, ISSN: 0268-9146, DOI: 10.1111/j.1365-2052.2004.01129.x
- KHLOPOVA N S ET AL: "Single nucleotide polymorphism of promoters of candidate genes controlling porcine productivity indices", RUSSIAN AGRICULTURAL SCIENCES, ALLERTON PRESS, INC, HEIDELBERG, vol. 38, no. 4, July 2012 (2012-07), pages 311-317, XP035122829, ISSN: 1934-8037, DOI: 10.3103/S106836741204009X
- DYAH MAHARANI ET AL: "Association of the gene encoding stearoyl-CoA desaturase (SCD) with fatty acid composition in an intercross population between Landrace and Korean native pigs", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 40, no. 1, 3 November 2012 (2012-11-03), pages 73-80, XP035150937, ISSN: 1573-4978, DOI: 10.1007/S11033-012-2014-0

## Description

### Field of the Invention

The present invention is comprised in the field of genetics, and more specifically under genetic methods for identifying pigs that have fats with a high monounsaturated fatty acid content based on the presence of genetic markers. The invention also relates to methods for breeding pigs that have fats with a high unsaturated fatty acid content.

### Background of the Invention

Animal fat is an essential component of meat for perceiving its texture, flavor and succulence. It is furthermore a source of fatty acids that cannot be synthesized by humans.

The proportion of fat in meat varies depending on the meat product, on the animal species and on the animal production system. The lipid fraction comprises between 12-20% of the weight of the pig ready for market. Lower values are generally a consequence of the breed or of commercial criteria.

There is a growing interest in determining the composition of the fat present in meat for human consumption that primarily comes from the current need in the industry to find methods that allow producing healthier meat. This is backed by the results obtained by several authors who relate the intramuscular fat composition with cardiovascular health, obesity or the risk of suffering carcinogenic diseases. It is therefore necessary to provide reliable methods that allow predicting the composition of the fat present in meat for human consumption.

Nutritional statements identify foods that are a source of nutrients that are beneficial for health that must be consumed quite frequently, as well as foods that are sources of nutrients that must be consumed less frequently as they are not healthy. As regards the fat content in foods, the nutritional regulation established in the European Community provides that a food has a high monounsaturated fat content if at least 45% of the fatty acids present in the product are from monounsaturated fat and if said fat provided more than 20% of the energy value of the product. Moderate consumption of monounsaturated fat is recommended by the World Health Organization due to the beneficial properties these fats confer, such as helping to control blood cholesterol levels (CINDI dietary guide, EUR/00/5018028), for example.

Muscular fat content and composition are two characteristics, among others, that define the quality of pork, especially that pork intended for the cured product market, hence the interest of companies improving porcine products and feed manufacturers in knowing how these characteristics evolve with the age and weight of the animal in order to control or modify them, whichever is appropriate.

The variety in fatty acid composition in adipose tissue and in muscle has very remarkable effects on the quality of the meat. The fatty acid composition determines the texture and the oiliness of adipose tissue as well as the oxidative stability of the muscle, which in turn affects the flavor and the color of the muscle.

A high polyunsaturated fatty acid (PUFA) content has adverse effects on the quality of the meat. Said effects are reflected both at the time of preserving the meat (due to lipid and myoglobin oxidation) and on the flavor thereof. However, a high monounsaturated fatty acid (MUFA) content is a sign of quality because these fatty acids favorably affect the organoleptic properties (visual appearance, texture, succulence and flavor) and nutritional properties of the meat. Furthermore, it has been verified in the case of pigs that it also has positive effects on the ham curing process.

The diet, the age of the animal, as well as genetic factors (for example, breed, sex or genotype), affect the fatty acid composition in tissues and therefore the quality of the meat. Porcine genetic enhancement programs have traditionally targeted enhancing reproductive and productive traits, among the latter of which those relating to carcass quality stand out.

The production of monounsaturated fats in pigs can be increased, for example, by means of modifying the diet, delaying the age at which the animal is sacrificed, or by castrating the animal. However, these techniques involve an increase in intramuscular fat, have a high cost and the result achieved in the animal is not transmitted to its descendents.

Physicochemical analysis of meat and its derivatives is a necessary method to assure product quality. Among the parameters that are analyzed, the percentage of fat present in the sample is determined by means of the method established in ISO Recommendation R-1443 (Official State Journal, 29/8/79) or variations thereof. This method, which allows determining the total fat percentage (in dry weight), consists of extracting fat from the sample using organic solvents that subsequently evaporate, finally weighing the obtained dry residue.

Quantitative analysis of fat in meat can also be carried out by means of near infrared spectrometry. This radiation is virtually absorbed by all the molecules, and the various components thereof can therefore be distinguished. Despite the fact that it is a fast technique that does not destroy the sample and requires little or no preparation for same, it is a methodology that does not allow being applied in a live animal (Bosch et al. 2009, Meat Science 82 (4) 432-437). Determination of the MUFA content in the fat of live animals must be done by means of a biopsy, a technique that is considered invasive, which affects the animal's wellbeing and is not recommended in routine analysis.

Uemoto Y. et al. (Animal Gen., 2011, 43: 225-228) have described different haplotypes associated with a higher MUFA content in pig muscular fat. In particular, two SNPs (g.-353C>T and g.-233T>C) were identified in the promoter region of the SCD gene as being completely linked, and shown to have a strong effect on fatty acid composition and melting point of fat.

Ren J. et al. (Animal Gen., 2004, 35: 255-257) also discloses known SNPs in the *SCD* gene, such as g.-353C>T and g.-233T>C.

KR20120072871 (A) discloses a method for predicting the content of unsaturated fatty acids in pigs by detecting one of SNP 1-27 or 29-42.

There is, however, a need in the state of the art to develop a method that allows improving the methods of determining MUFA levels in live animals to be applied to processes for selecting, breeding and classifying quality market animals or animals for producing meat derivatives.

### Brief Description of the Invention

The authors of the present invention have identified the existence of a polymorphic position in the promoter region of the porcine stearoyl-coenzyme A desaturase gene (*SCD*). Specifically, it is at position 2281 based on the numbering of the sequence AY487830 in the NCBI database from September 15, 2004 (corresponding to position -180 with respect to the translation start codon), which can be occupied by a "G" or by an "A" (SNP3 polymorphism (2281G/A)). The authors of the present invention have additionally found that surprisingly, the presence of an A in at least one allele of said polymorphic position of the *SCD* gene is correlated in a statistically significant manner with high levels of monounsaturated fatty acids (MUFA) in the intramuscular fat of animals (see Examples 1-3 of the present invention). Said change in the percentage of MUFA is not accompanied by changes in weight of the carcass or of the animal, or in the percentage of lean meat of the carcass or intramuscular fat (IMF) (see Example 3 of the present invention). The authors of the present invention have observed that said effect is also produced in pigs having other polymorphisms in the *SCD* gene, specifically T/C polymorphism at position 2228 and A/G polymorphism at position 15109 (using in both cases the numbering of the sequence defined by accession number AY487830 in the NCBI database).

These findings allow developing non-aggressive, fast and reliable methods that allow evaluating both in live animals and in the meat products derived from said animals if said animals are producers of high levels of MUFA.

### Detailed Description of the Invention

### Polynucleotides comprising the polymorphic position at position -180 of the SCD gene

The present invention therefore relates to the identification of an SNP in the porcine *SCD* gene and a method that allows evaluating if the animals contain high levels of MUFA based on the genotype of SNPs in the *SCD* gene.

In a first aspect, it is disclosed an isolated polynucleotide comprising a region of at least 10 consecutive nucleotides of a polymorphic variant of the *Sus scrofa domestica SCD* gene, characterized by the presence of an A at position 2281 based on the numbering of the sequence defined by accession number AY487830 in the NCBI database, and wherein said region comprises the nucleotide at position 2281, or a polynucleotide complementary to said polynucleotide.

As it is used herein, the expression "polynucleotide", refers to a genomic DNA or RNA fragment, where said DNA or RNA can be single-stranded or double-stranded and can represent a sense or antisense strand, peptide nucleic acid (PNA) or any natural DNA or RNA type material. As persons skilled in the art will understand, when the nucleic acid is RNA, deoxynucleotides A, G, C and T are replaced with ribonucleotides A, G, C and U, respectively. In one disclosure, the polynucleotide comprises a DNA fragment comprising at least 10 nucleotides, at least 15 nucleotides, at least 20 nucleotides, at least 25 nucleotides, at least 30 nucleotides, at least 40 nucleotides, at least 50 nucleotides, at least 60 nucleotides, at least 70 nucleotides, at least 80 nucleotides, at least 90 nucleotides, at least 100 nucleotides, all of said nucleotides being consecutive, of the polymorphic variant of the *Sus scrofa domestica SCD* gene.

The term "isolated" refers to material that is substantially or essentially free of components that usually accompany the material as it is found in its native state. Typically, the isolated nucleic acids are at least about 50%, 55%, 60%, 65%, 70%, 75%, 80% or 85% pure, usually at least about 90%, 91%, 92%, 93%, 94%, 95%, 96%, 97%, 98% or 99% pure. The purity or homogeneity can be indicated by a number of means that are well known in the art, such as polyacrylamide gel electrophoresis of a nucleic acid sample, followed by viewing after staining. For some purposes, high resolution will be needed and HPLC or similar means will be used for purification. For oligonucleotides or other products sialylated, the purity can be determined using, for example, thin layer chromatography, HPLC or mass spectroscopy.

As it is used herein, the expression "*Sus scrofa domestica* stearoyl coA desaturase (*SCD*) gene" refers to the gene encoding the stearoyl coA desaturase protein. The sequence of the Sus scrofa *SCD* gene is deposited in the GenBank database (version from September 15, 2004) under accession number AY487830.1 (SEQ ID NO:1). The sequence of the coding region of said gene (corresponding to regions 2461 to 2487, 3006 to 3288, 8073 to 8203, 9562 to 9767, 11236 to 11468 and 14423 to 14622 of the *SCD* gene described in the sequence AY487830.1) corresponds to the sequence with accession number NM_213781.1 in the NCBI database. The protein encoded by said gene is defined in the UniProtKB/Swiss-Prot database with accession number O02858. This protein participates in the long-chain fatty acid unsaturation pathway by catalyzing the synthesis of monounsaturated fatty acids from saturated fatty acids. The principal *SCD* reaction product is oleic acid, which is obtained by means of stearic acid unsaturation.

As it is used herein, the expressions "polymorphic variant" or "allelic variant" are used indistinctly to refer to an alternative form of a gene, wherein a variable number of positions in said gene are occupied by a nucleotide that is an alternative to the nucleotide in said gene. Polymorphic variants are distinguished from one another by the presence of different nucleotides at points referred to as SNPs or "single nucleotide polymorphisms".

The term "single nucleotide polymorphism (SNP)" refers to a polymorphic site occupied by a single nucleotide, which is the site of the variation between the allelic sequences. The site is usually preceded and followed by highly conserved sequences of the allele (for example, the sequences varying by less than 1/100 or 1/1000 members of a population). An SNP usually arises due to the substitution of one nucleotide with another in the polymorphic site. SNPs can also arise from a nucleotide deletion or a nucleotide insertion in relation to a reference allele. The polymorphic site is typically occupied by a base other than the reference base. For example, when the reference allele contains the "T" (thymidine) base in the polymorphic site, the altered allele can contain a "C" (cytidine), "G" (guanine), or "A" (adenine) in the polymorphic site. SNPs can appear in the coding region of nucleic acids, in which case they can give rise to a variant or even defective protein. These SNPs can alter the coding sequence of the gene and therefore specify another amino acid (a missense SNP), or they can introduce a stop codon (a nonsense SNP). When an SNP does not alter the amino acid sequence of a protein, the SNP is called a "silent" SNP. SNPs can also appear in the non-coding regions of the nucleotide sequence, including in the promoter region, in introns, in 5'-UTR regions and/or in 3'-UTR regions. When the change in the nucleotide corresponds with a change in the amino acid in the protein, the polymorphism can be named as "aa1-nn-aa2", where "aa1" represents the original amino acid, "nn" corresponds to the position of the mutated amino acid, and "aa2" represents the amino acid resulting from the mutation. However, when the change in the nucleotide does not correspond with a change in the corresponding amino acid, the polymorphism is named so as to indicate the position of the nucleotide that contains the mutation and the change in the nucleotide is indicated as "mm nt1 > nt2", wherein "mm" indicates the number of the nucleotide in which the change has occurred, "nt1" represents the original nucleotide and "nt2" represents the changed nucleotide. SNPs generally represent one of the most common forms of genetic variations and usually occur when a single nucleotide is altered in the genome (for example, through substitution, addition or deletion). Each version of the sequence with respect to the polymorphic site is known as an allele of the polymorphic site. SNPs occur throughout the entire genome, both in extragenic regions and in coding regions or in their regulatory sequences. SNPs tend to be evolutionarily stable from generation to generation and, as such, they can be used to study specific genetic variations in the population. Some SNPs can be in non-coding regions, for example, in regulatory regions, where their possible functional effects can be very important because they can give rise to differential or advantageous or defective splicing or to alterations in protein expression levels. SNPs can therefore serve as effective indicators of protein expression levels or of the activity of said proteins.

As it is used herein, the term "allele" refers to one, two or more forms of a gene, locus or genetic polymorphism. Sometimes different alleles can give rise to different phenotypes; however, other times different alleles will have the same result in the expression of a gene. Most multicellular organisms have two sets of chromosomes, i.e., they are diploid. These chromosomes are referred to as homologous chromosomes. Diploid organisms have a copy of each gene (and an allele) in each chromosome. If both alleles are the same, they are homozygotes. If the alleles are different, they are heterozygotes.

The polymorphic variant from which the polynucleotide is derived is the variant at position 2281 based on the numbering of the sequence defined by accession number AY487830 in the NCBI database, which corresponds to position -180 with respect to the start codon of the *Sus scrofa SCD* gene, the adenine of said start codon being the nucleotide at position 1. The polymorphic variant contains a G in said position. *Probes and kits suitable for detecting polymorphisms associated with a high monounsaturated fatty acid content*

In another aspect, it is disclosed a probe capable of detecting the nucleotide at position 2281 of the *S. scrofa domestica SCD* gene based on the numbering of the sequence defined by accession number AY487830 in the NCBI database in the *Sus scrofa domestica SCD* gene or in a polymorphic variant at position 2281 of said sequence.

The probes are capable of hybridizing in a specific manner with a nucleic acid containing a specific allele in a specific polymorphic position without showing detectable hybridization to the nucleic acid containing another allele in said polymorphic position. The present disclosure thus relates to probes that are capable of hybridizing in a specific manner with the polymorphic variant of the *SCD* gene in which the polymorphic position at position 2281 based on the numbering of the sequence defined by accession number AY487830 in the NCBI database is occupied by a G without showing detectable hybridization to the polymorphic variant of the *SCD* gene in which the polymorphic position at position 2281 based on the numbering of the sequence defined by accession number AY487830 in the NCBI database is occupied by an A. Alternatively, the present disclosure refers to probes which are capable of hybridizing in a specific manner with the polymorphic variant of the *SCD* gene in which the polymorphic position at position 2281 based on the numbering of the sequence defined by accession number AY487830 in the NCBI database is occupied by an A without showing detectable hybridization to the polymorphic variant of the *SCD* gene in which the polymorphic position at position 2281 based on the numbering of the sequence defined by accession number AY487830 in the NCBI database is occupied by a G.

As it is used herein, the term "probe" refers to an oligonucleotide having a defined sequence capable of hybridizing in a specific manner with a complementary sequence of a nucleic acid, so it can be used for detecting and identifying complementary or substantially complementary sequences in nucleic acids. The length of the probe can vary within a broad range although, for practical reasons, probes having a short length comprised between 15 bases and 30 bases, preferably between 16 bases and 22 bases, are preferred. The use of probes having a longer or shorter length would not affect the sensitivity or specificity of the technique, but it would require performing a series of modifications of the conditions on which same is performed by varying melting temperature thereof and their GC content, which would fundamentally affect the hybridization temperature and time.

Likewise, as it is used herein, the term "hybridization" refers to the formation of a duplex type structure by two nucleic acids due to complementary base pairing. Hybridization can occur between completely complementary nucleic acids chains or between "substantially complementary" nucleic acid chains containing small mismatched regions. The conditions under which completely complementary nucleic acid chains hybridize are referred to as "strict hybridization conditions" or "sequence-specific hybridization conditions". Nevertheless, substantially complementary stable double nucleic acid chains can be obtained under less strict hybridization conditions, in which case the tolerated degree of mismatching can be adjusted by means of a suitable adjustment of the hybridization conditions. The person skilled in the art can empirically determine the stability of a duplex taking into account a number of variables, such as the length and concentration of base pairs of the probes, ionic strength and the incidence of mismatched base pairs, following the guidelines from the state of the art (see, for example, Sambrook et al., Molecular Cloning: A Laboratory Manual, Cold Spring Harbor Laboratory, Cold Spring Harbor, N.Y. (1989); and Wetmur, Critical Reviews in Biochem. and Mol. Biol. 26 (3/4) :227-259 (1991)).

The allele-specific probes according to the present disclosure are thus capable of specifically hybridizing to the polymorphic variants of the *SCD* gene characterized by the nucleotide at position 2281 of said gene based on the numbering of the sequence defined by accession number AY487830 in the NCBI database.

In the context of the present disclosure, specific hybridization is understood as the capability of a first nucleic acid to bind to, form a duplex or hybridize with a second nucleic acid such that the second nucleic acid can be identified or distinguished from other components of a mixture (for example, cell extracts, genomic DNA, etc.). Specific hybridization may be carried out under highly restrictive conditions or under moderately restrictive conditions.

Typical highly restrictive hybridization conditions include incubation in 6 X SSC (1 X SSC: 0.15 M NaCl, 0.015 M sodium citrate) and 40% formamide at 42°C for 14 hours, followed by one or several washing cycles using 0.5 X SSC, 0.1% SDS at 60°C. Alternatively, highly restrictive conditions include those comprising hybridization at a temperature of about 50°-55°C in 6 X SSC and a final washing at a temperature of 68°C in 1-3 X SSC. Moderately restrictive conditions comprise hybridization at a temperature of about 50°C up to about 65°C in 0.2 or 0.3 M NaCl, followed by washing at about 50°C up to about 55°C in 0.2 X SSC, 0.1% SDS (sodium dodecyl sulfate).

In a particular disclosure, the probe is capable of detecting nucleotide G at position -180 of the *S. scrofa domestica SCD* gene. In a more particular disclosure, said probe has the nucleotide sequence of SEQ ID NO: 16.

In another particular disclosure, the probe is capable of detecting nucleotide A at position -180 of the *S. scrofa domestica SCD* gene. In another more particular disclosure, said probe has the nucleotide sequence of SEQ ID NO: 17.

The probes are complementary or substantially complementary to specific target sequences contained in the *S. scrofa domestica SCD* gene.

The probes will have high homology with the specific target sequences contained in the *S. scrofa domestica SCD* gene. "High homology" usually refers to a 40% homology or greater, preferably 60% homology or greater, more preferably 80% homology or greater, even more preferably 95% homology or greater. Probe homology can be determined following the algorithm in Wilbur and Lipman, 1983, Proc Natl. Acad. Sci. USA, 80: 723-30.

Optionally, if desired, the probes can be labeled for the purpose of being able to subsequently detect the amplified fragments. The labeling can be performed by conventional methods. Said labeling can be direct, for which fluorophores, for example, Cy3, Cy5, fluorescein, alexa, etc., enzymes, for example, alkaline phosphatase, peroxidase, etc., radioactive isotopes, for example, 33P, 1251, etc., or any other label known by the person skilled in the art can be used. Alternatively, said labeling can be indirect by means of using chemical methods, enzymatic methods, etc.; by way of illustration, the amplification product can incorporate a member of a specific binding pair, for example, avidin or streptavidin conjugated with a fluorochrome (locus), and the probe binds to the other member of the specific binding pair, for example, biotin (indicator), the reading being performed by means of fluorimetry, etc.

Thus in a particular disclosure, the labeling of the probe is carried out by means of labeling at one of its ends; in another even more particular disclosure, the compound used in labeling the probe is selected from the group consisting of a radioisotope, a fluorescent material, digoxigenin and biotin. Examples of fluorescent materials that can be used in labeling oligonucleotides include, but are not limited to, 5-carboxyfluorescein (5-FAM), 6-FAM, tetrachlorinated analogue of t-FAM (TET), hexachlorinated analogue of 6-FAM (HEX), 6-carboxytetramethylrhodamine (TAMRA), 6-carboxy-X-rhodamine (ROX), 6-carboxy-4',5'-dichloro-2',7'-dimethoxyfluorescein (JOE), NED, Cy-3, Cy-5, Cy-5.5, fluorescein-6-isothiocyanate (FITC), VIC, and tetramethylrhodamine-5-isothiocyanate (TRITC).

In a more particular disclosure, the labeling of the probe that is capable of detecting nucleotide A at position -180 of the *S. scrofa domestica SCD* gene is carried out with the fluorescent compound 5-carboxyfluorescein (5-FAM).

In another more particular disclosure, the labeling of the probe that is capable of detecting nucleotide G at position -180 of the *S. scrofa domestica SCD* gene is carried out with the fluorescent compound VIC.

The present disclosure also contemplates kits, comprising the probe disclosed herein above.

In a preferred disclosure, the kit comprises a probe capable of detecting the nucleotide at position 2281 of the *S. scrofa domestica SCD* gene based on the numbering of the sequence defined by accession number AY487830 in the NCBI database in the *Sus scrofa domestica SCD* gene or in a polymorphic variant at position 2281 of said sequence. In a preferred disclosure, the probe is suitable for the detection of the variant of the *S. scrofa domestica SCD* gene based on the numbering of the sequence defined by accession number AY487830 in which the nucleotide occupying position 2281 of the *SCD* gene is a G nucleotide. In a more preferred disclosure, the kit comprises a probe comprising the sequence of SEQ ID NO:16.

In another preferred disclosure, the probe is suitable for the detection of the variant of the *S. scrofa domestica SCD* gene based on the numbering of the sequence defined by accession number AY487830 in which the nucleotide occupying position 2281 of the *SCD* gene is an A nucleotide. In a more preferred disclosure, the kit comprises a probe comprising the sequence of SEQ ID NO:17.

In a disclosure, the kits additionally comprise probes suitable for the detection of other polymorphisms which are associated with the unsaturated fatty acid content in the intramuscular fat of pigs. Thus in another preferred disclosure, the kit additionally comprises a probe for the detection of the nucleotide at position 2228 and/or a probe for the detection of the nucleotide at position 15109 in the *SCD* gene based on the numbering of the sequence defined by accession number AY487830 in the NCBI database.

Position 2228 in the *SCD* gene corresponds to position -233 with respect to the start codon of the *Sus scrofa SCD* gene, the adenine of said start codon being the nucleotide at position 1. Said position can contain a T or a C.

Position 15109 in the *SCD* gene is located in the non-coding 3' region of the *SCD* gene and corresponds to position 1566 with respect to the start codon of the *Sus scrofa SCD* gene, the adenine of said start codon being the nucleotide at position 1. Said position can contain a G or an A.

In a particular disclosure, the probes forming part of the kit are labeled at one of their ends with a fluorescent compound. In a preferred disclosure, said fluorescent compound is selected from the group consisting of a radioisotope, a fluorescent material, digoxigenin and biotin. Examples of fluorescent materials that can be used in labeling oligonucleotides include, but are not limited to, 5-carboxyfluorescein (5-FAM), 6-FAM, tetrachlorinated analogue of t-FAM (TET), hexachlorinated analogue of 6-FAM (HEX), 6-carboxytetramethylrhodamine (TAMRA), 6-carboxy-X-rhodamine (ROX), 6-carboxy-4',5'-dichloro-2',7'-dimethoxyfluorescein (JOE), NED, Cy-3, Cy-5, Cy-5.5, fluorescein-6-isothiocyanate (FITC), VIC, and tetramethylrhodamine-5-isothiocyanate (TRITC).

The disclosure additionally relates to primers that allow amplifying in a specific manner the region of the *Sus scrofa SCD* gene (accession number AY487830 in the NCBI database) comprising the polymorphic position at position 2281. Thus in another aspect, the disclosure relates to a kit for detecting the polymorphism at position 2281 of the *S. scrofa domestica SCD* gene based on the numbering of the sequence defined by accession number AY487830 in the NCBI database comprising a first and a second primer complementary to sequences in the *SCD* gene at position 5' and 3' with respect to said position.

It is disclosed that the first primer comprises the sequence defined in SEQ ID NO:14 and/or wherein the second primer comprises the sequence defined in SEQ ID NO:15.

Additionally, the kit can comprise additional primer pairs that allow amplifying in a specific manner the regions of the *SCD* gene wherein other polymorphic positions that are associated with the unsaturated fatty acid content in the intramuscular fat of pigs are located. Thus in another aspect, the disclosure relates to a kit additionally comprising a second primer pair, wherein the first and second primer of said second primer pair are complementary to sequences in the *SCD* gene at position 5' and 3' with respect to position 2228 of said gene and/or a third primer pair, wherein the first and second primer of said third primer pair are complementary to sequences in the *SCD* gene at position 5' and 3' with respect to position 15109 based on the numbering of the sequence defined by accession number AY487830 in the NCBI database.

Materials suitable for being included in an exemplary kit according to the present disclosure comprise one or more of the following: primer pairs specific for PCR (oligonucleotides) hybridizing to domains of the DNA or cDNA sequence flanking the genetic polymorphisms of interest (SNPs); reagents capable of amplifying a domain of a specific sequence, whether in genomic DNA or cDNA without having to perform PCR; reagents necessary for discriminating between different alleles in the sequence of the domains amplified by PCR and non-PCR amplification (for example, restriction endonucleases, oligonucleotides preferably hybridizing to an allele of the polymorphism, including those modified for containing enzymes or fluorescent chemical groups that amplify the signal of the oligonucleotides and make discrimination of the alleles more robust); reagents necessary for physically separating the products derived from the different alleles (for example agarose or polyacrylamide and a buffer for being used in the electrophoresis, HPLC columns, SSCP gels, formamide gels or a MALDI-TOF matrix support).

The document discloses the use of the kit to determine if a subject has at least one SNP of the *SCD* gene associated with a high degree of fatty acid unsaturation. In a particular disclosure, the degree of unsaturation refers to the C18:1/C18:0 fatty acid ratio.

The document discloses the use of the kit in a subject, preferably in a pig. In a more particular disclosure, the kit refers to a pig from the Duroc breed.

### Genotyping methods

In one aspect, the invention relates to a genotyping method, hereinafter, "first method of the invention", for detecting the nucleotide occupying position 2281 in the *S. scrofa domestica SCD* gene based on the numbering of the sequence defined by accession number AY487830 in the NCBI database.

Methods which are known in the state of the art for genotyping a polymorphism and can be used in the context of the present invention include, but are not limited to, any of the methods described by Rapley and Harbron, (Molecular Analysis and Genome Discovery. Chichester. John Wiley and Sons Ltd. 2004) such as:
- Methods based on hybridization: Dynamic allele-specific hybridization (DASH), DNA microarrays, molecular beacons,
- Methods based on enzymes such as DNA ligase, DNA polymerase and nuclease. Examples of said methods include allele-specific PCR, invader assay (Flap endonuclease), single base extension (SBE), TaqMan (5'-3'-endonuclease), restriction fragment length polymorphisms (RFLP), direct sequencing and ligation assay.
- Post-amplification methods based on physical properties of DNA such as the melting temperature of DNA hybrids and single chain conformation. Such methods include single chain conformation polymorphism (SSCPs), temperature gradient gel electrophoresis (TGGE), denaturing high performance liquid chromatography (DHPLC).

The genotyping method according to the present invention can obviously be carried out starting from any nucleic acid strand or from both strands. In a particular embodiment, SNPs are detected by means of the allelic discrimination technique. This methodology allows detecting variations in the DNA sequence, for example SNPs, by means of reading the fluorescence emitted by each of the complementary probes used for each of the SNPs since they are labeled with different fluorochromes. In a more particular embodiment, genotyping is performed by means of the aforementioned allelic discrimination method coupled to a real-time PCR apparatus.

In a preferred embodiment, the genotyping method of the present invention additionally comprises the detection of other polymorphisms in the *SCD* gene which are associated with the unsaturated fatty acid content in the intramuscular fat of pigs. Thus in a preferred embodiment, the genotyping method according to the present invention additionally comprises detecting the nucleotide occupying position 2228 of said gene and/or the nucleotide occupying position 15109 in the *S. scrofa domestica SCD* gene based on the numbering of the sequence defined by accession number AY487830 in the NCBI database.

In a preferred embodiment, genotyping is carried out by means of hybridization to allele-specific probes capable of detecting the nucleotide at position 2281 of the *S. scrofa domestica SCD* gene based on the numbering of the sequence defined by accession number AY487830 in the NCBI database in the *Sus scrofa domestica SCD* gene the gene or in a polymorphic variant at position 2281 of said sequence. In a more preferred embodiment, the allele-specific probe comprises the sequence of SEQ ID NO:16 and allows detecting variants of the *SCD* gene, wherein the nucleotide occupying position 2281 of the *SCD* gene is a G nucleotide. In another preferred embodiment, the allele-specific probe comprises the sequence of SEQ ID NO:17 and allows detecting variants of the *SCD* gene, wherein the nucleotide occupying position 2281 of the *SCD* gene is an A nucleotide. The probes are preferably labeled at one of their ends with a fluorescent compound. In an even more preferred embodiment, the fluorescent compound is VIC or 5-carboxyfluorescein (5-FAM).

In another preferred embodiment, the genotyping method according to the present invention comprises amplification of the region or regions of the *S. scrofa domestica SCD* gene containing the polymorphic position or the polymorphic positions to be characterized. In the case of the polymorphism at position 2281, in a preferred embodiment amplification of the region is carried out using a primer pair, wherein the first primer comprises the sequence defined in SEQ ID NO:14 and/or wherein the second primer comprises the sequence defined in SEQ ID NO:15.

### Methods for predicting the degree of fatty acid unsaturation

In another aspect, the invention relates to a method, hereinafter, "second method of the invention", for predicting the degree of fatty acid unsaturation in a subject or a meat derivative of said subject, which comprises detecting in a sample from said subject the nucleotide of at least one polymorphic position in the *Sus scrofa domestica SCD* gene, wherein said polymorphic position is position 2281 based on the numbering of the sequence defined by accession number AY487830 in the NCBI database and wherein the presence of nucleotide A at position 2281 in at least one allele of the *SCD* gene is indicative of a high degree of fatty acid unsaturation with respect to the reference value.

In a particular embodiment, the method additionally comprises detecting in said sample the nucleotide occupying position 2228 of said gene and/or the nucleotide occupying position 15109 in the S. scrofa domestica SCD gene based on the numbering of the sequence defined by accession number AY487830 in the NCBI database.

The method of the invention comprises determining the presence or absence of the alleles associated with a specific condition, in this particular case, a high degree of fatty acid unsaturation. The alleles associated with a high degree of unsaturation are referred to as beneficial alleles and are shown in Table 1.

**Table 1: List of identified beneficial alleles. The column "Position in the Sus scrofa SCD gene" comprises the position with respect to the transcription start codon followed by the absolute position in the sequence AY487830 in the NCBI database.**

| POLYMORPHISM NAME | POSITION IN *S. scrofa SCD* GENE | CHANGE IN NUCLEOTIDE | BENEFICIAL ALLELE |
|---|---|---|---|
| SNP2 | -233/2228 | T>C | T |
| SNP3 | -180/2281 | A>G | A |
| SNP5 | +1566/15109 | A>G | A |

As it is used herein, the term "predicting" refers to the determination of the probability that the fat from the subject has a specific degree of unsaturation. As the persons skilled in the art will understand, such determination does not usually seek to be correct for all (i.e., 100%) of the subjects that are going to be identified. However, the term requires that a statistically significant part of the subjects can be identified (for example, a cohort in a cohort study). The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

As it is used herein, the term "associated with" means that a particular polymorphic site is structurally and/or functionally associated with a specific SNP, for example, said polymorphic site is in the corresponding locus, or in the immediate proximity thereof, and/or said polymorphic site interacts with or affects the corresponding gene or the expression of the product thereof.

As it is used herein, the expression "degree of fatty acid unsaturation" refers to the proportion of one or several MUFAs present in a sample of a subject with respect to the proportion of the total fatty acids present in said sample from said subject. In an even more preferred embodiment, the degree of unsaturation corresponds to the percentage of oleic acid with respect to the total fatty acids in the sample. In another embodiment, the degree of unsaturation corresponds to the percentage of oleic acid with respect to stearic acid (C18:0) in the sample.

As it is used herein, the expression "fatty acids" refers to lipid biomolecules formed by a long linear hydrocarbon chain, with a different length or number of carbon atoms having an alkyl group at one end and an acid group at the other end. Fatty acids can be classified as saturated fatty acids and unsaturated fatty acids. Saturated fatty acids do not have double bonds in the hydrocarbon chain forming them and are flexible and solid at room temperature, whereas unsaturated fatty acids have double or triple bonds, are rigid at the level of these bonds and are liquid or viscous at room temperature. In this group, distinction can further be made between monounsaturated fatty acids or MUFAs and polyunsaturated fatty acids or PUFAs. In the nomenclature used for naming the fatty acids of the invention, reference is first made to the total number of carbons of the hydrocarbon molecule, followed by a colon and a number referring to the number of double bonds found in said chain, and finally the term "n-number" refers to the position that the first double bond occupies with respect to the carbon of the methyl group (CH₃).

As it is used herein, the term "MUFA" refers to fatty acids with an even-numbered carbon chain containing a single double bond in their structure, i.e., a single carbon-carbon double bond (-CH=CH-). Examples of MUFA are palmitoleic acid (16:1 n-7), cis-vaccenic acid (18:1 n-7), eicosanoic acid (20:1 n-9), erucic acid (22:1 n-9), nervonic acid (24:1 n-9) and oleic acid (18:1 n-9).

As it is used herein, the term "PUFA" refers to fatty acids whose carbon chain has more than one double bond. Examples of PUFA are hexadecatrienoic acid (16:3 n-3), alpha-linolenic acid (18:3 n-3), stearidonic acid (18:4 n-3), eicosatrienoic acid (20:3 n-3), eicosatetraenoic acid (20:4 n-3), eicosapentaenoic acid (20:5 n-3), docosapentaenoic acid (22:5 n-3), tetracosapentaenoic acid (24:5 n-3), tetracosahexaenoic acid (24:6 n-3), linoleic acid (18:2 n-6), gamma-linolenic acid (18:3 n-6), eicosadienoic acid (20:2 n-6), arachidonic acid (22:4 n-6), docosadienoic acid (22:2 n-6), adrenic acid (22:4 n-6), docosapentaenoic acid (22:5 n-6), tetracosatetraenoic acid (24:4 n-6) and tetracosapentaenoic acid (24:5 n-6).

As it is used herein, the term "sample" refers to any sample that can be obtained from a subject in which there is genetic material that allows determining the nucleotide in the polymorphic positions indicated above. Biological samples suitable for use in the present invention include, for example, a biopsy sample, a sample of a meat derivative, a biological fluid (for example, blood, plasma, sperm, serum, urine, etc.), a cell, a tissue (for example adipose tissue, muscle tissue, epithelial tissue, etc.), etc. The samples can be obtained by conventional methods, for example, scraping, biopsy, etc., using methods that are well known by the persons skilled in the art. In a particular embodiment, the sample is a nucleic acid sample, for example: a sample comprising a nucleic acid, for example, DNA, genomic DNA (gDNA), complementary DNA (cDNA), RNA, heterogeneous nuclear RNA (hnRNA), mRNA, etc., of the subject. In one embodiment, the sample is isolated or extracted from a particular cell or tissue. The cells can be present in a biological fluid, for example, blood, saliva, sperm, etc., in a tissue, for example muscle or adipose tissue, etc. In a particular embodiment, the sample is a biological fluid, such as saliva, blood, sperm or the like. In a preferred embodiment, the sample in which the determination of the presence of the different polymorphisms is carried out is a muscle sample. In a preferred embodiment, the muscle is selected from the gluteus medius muscle of the ham, the longissimus dorsi muscle and the semimembranosus muscle. Methods for the isolation of cell and tissue samples are well known by the persons skilled in the art and include, but are not limited to, aspirations, tissue sections, needle biopsies, etc.

As it is used herein, the term "subject" refers to any animal classified as a mammal. The subject is preferably a pig. As it is used herein, the term "pig" refers to any domestic or wild species belonging to the Sus genus, including *Sus barbatus, Sus bucculentus, Sus cebifrons, Sus celebensis, Sus domestica, Sus falconeri, Sus heureni, Sus hysudricus, Sus philippensis, Sus salvanius, Sus scrofa, Sus strozzi, Sus timoriensis, Sus verrucosus.* In a preferred embodiment, the determination is carried out in the common pig or *S. scrofa domestica.* Synonyms that can be used to name a pig are, for example, hog, swine, boar, shoat, porky, piggy, sow, piglet, porker, cob roller, etc. As it is used herein, the term pig encompasses any pig breed, variety or stock. Examples of domestic pig subspecies that can be analyzed by means of the method of the present invention are: Castilian wild boar (*Sus scrofa castilianus*), Iberian wild boar *(Sus scrofa baeticus), Sus scrofa scrofa, Sus scrofa meridionalis, Sus scrofa majori, Sus scrofa attila, Sus scrofa ussuricus, Sus scrofa cristatus, Sus scrofa vittatus, Sus scrofa taivanus.* According to the present invention, the term "breed" refers to a group of individuals of the same species having the same differentiated qualities, aptitudes or traits, and they pass them on from generation to generation. Examples of breeds of domestic pigs that can be analyzed by means of the method of the present invention are: *Hampshire, Pietrain, Belgian Landrace, Meishan, Berkshire, Large White, Landrace* or *Duroc.* A variety referring to a group of individuals of the same breed having an inheritable differentiated trait or aptitude, which is usually the color, can be defined within a breed. A variety can contain different stocks referred to as a group of individuals of the same breed and variety that reproduce without the intervention of foreign contributions and in a number of specimens that is enough to keep inbreeding rather low. In a preferred embodiment, the subject is a pig from the Duroc breed.

In a particular embodiment, the method of the invention allows determining the degree of unsaturation in the muscle of the gluteus medius of the ham, the longissimus dorsi muscle and/or the semimembranosus muscle.

In the particular case of wanting to determine the degree of unsaturation in the fat present in a meat derivative of a subject, the sample used for the determination of polymorphisms is a sample of said meat derivative.

As it is used herein, the expression "meat derivative" refers to a food product that is entirely or partially prepared with meats or scraps of the species authorized for such purpose, and subjected to specific operations for preservation before being made available for consumption. Within the scope of the present invention, included among meat derivatives are raw and cured meat products, cured meat products being those which are subjected to a maturation or drying process, either ground and stuffed (various types of sausage, i.e., *chorizo, salchichón, chorizo blanco, chorizo Pamplona, salami, morcón, fuet,* etc.) or whole (cured ham, cured shoulder, belly pork, bacon, *cecina* (dried meat), dry-cured pork loin, etc.). Heat-treated meat products are those obtained by heat treatment, which are either ground and stuffed (Frankfurt type wieners, mortadella, bologna, olive loaf, etc.) or whole (cooked ham, cooked shoulder, cooked lean pork, cold cuts, etc.). Fresh meat products are those prepared without being subject to drying treatment or to heat treatment. They can be ground and stuffed (fresh sausage, fresh *chorizo,* etc.), only ground (ground meat, hamburgers, meat pies, etc.) or whole (marinated loin). Such products behave similarly to fresh meat as regards preservation. In a more particular embodiment, the sample is obtained from ham and/or loin.

The detection of the polymorphism or polymorphisms according to the second method of the invention can be carried out using any of the genotyping methods described in the context of the first method of the invention. In a preferred embodiment, genotyping is carried out by means of hybridization to allele-specific probes capable of detecting the nucleotide at position 2281 of the *S. scrofa domestica SCD* gene based on the numbering of the sequence defined by accession number AY487830 in the NCBI database in the *Sus scrofa domestica SCD* gene or in a polymorphic variant at position 2281 of said sequence. In a more preferred embodiment, the allele-specific probe comprises the sequence of SEQ ID NO:16 and allows detecting variants of the *SCD* gene, wherein the nucleotide occupying position 2281 of the *SCD* gene is a G nucleotide. In another preferred embodiment, the allele-specific probe comprises the sequence of SEQ ID NO:17 and allows detecting variants of the *SCD* gene, wherein the nucleotide occupying position 2281 of the *SCD* gene is an A nucleotide. The probes are preferably labeled at one of their ends with a fluorescent compound. In an even more preferred embodiment, the fluorescent compound is VIC or 5-carboxyfluorescein (5-FAM).

In another preferred embodiment, the genotyping method according to the present invention comprises amplification of the region or regions of the *S. scrofa domestica SCD* gene containing the polymorphic position or the polymorphic positions to be characterized. In the case of the polymorphism at position 2281, in a preferred embodiment, amplification of the region is carried out using a primer pair, wherein the first primer comprises the sequence defined in SEQ ID NO:14 and/or wherein the second primer comprises the sequence defined in SEQ ID NO:15.

According to the second method of the invention, the presence of nucleotide A at position 2281, of nucleotide T at position 2228 and/or of nucleotide A at position 15109 in at least one allele of the *SCD* gene is associated with a high degree of unsaturation in the intramuscular fat of an animal. The degree of unsaturation is related to the number of favorable alleles. Thus in the case of the polymorphism at position 2281, A allele homozygous animals show a higher degree of unsaturation in fat that AG heterozygous animals, and the latter show a higher degree of unsaturation in fat than GG homozygous animals.

As it is used herein, the expression "high degree of unsaturation" refers to the values of the degree of unsaturation being high with respect to reference values for said degree of unsaturation. According to the present invention, it is considered that the degree of unsaturation is high with respect to a reference value when the levels in the sample of the subject are increased by at least 0.8%, at least 0.85%, at least 0.9%, at least 0.95%, at least 1%, at least 1.5% , at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90%, at least 100% or more with respect to a reference value for said parameter.

Reference values for the degree of unsaturation that can be used in the context of the present invention include, for example, the degree of unsaturation in animals having one or more markers associated with a low degree of unsaturation. Thus in a preferred embodiment, the degree of unsaturation of intramuscular fat or of subcutaneous fat obtained from a pig which is homozygous for nucleotide G at position 2281 of the *SCD* gene, the degree of unsaturation of the intramuscular fat obtained from a pig which is homozygous for nucleotide C at position 2228 and/or the degree of unsaturation of the intramuscular fat obtained from a pig which is homozygote for nucleotide G at position 15109 is used as the reference value. Alternatively, the degree of unsaturation of intramuscular or subcutaneous fat obtained from a pig which is homozygous for several of the polymorphic positions correlated with a low unsaturation index can be used as the reference value.

Furthermore, the inventors have also observed that when the subject has in the SNP according to the present invention the nucleotide indicative of a high degree of fatty acid unsaturation (beneficial allele) in the two alleles (i.e., the subject is homozygous for the SNP associated with a high degree of fatty acid unsaturation), the degree of fatty acid unsaturation with respect to a subject having said beneficial allele in only one of the alleles of the gene is increased. Therefore in another particular embodiment, the method of the invention involves detecting in the SNP, the nucleotide in at least one polymorphic position in the *Sus scrofa domestica SCD* gene, wherein said polymorphic position is position 2281 based on the numbering of the sequence defined by accession number AY487830 in the NCBI database, and wherein the presence in the two alleles of the *SCD* gene of nucleotide A at position 2281 is indicative of a high degree of fatty acid unsaturation with respect to the reference value. In yet another particular embodiment, the method additionally comprises detecting in said sample the nucleotide occupying position 2228 of said gene and/or the nucleotide occupying position 15109 in the *S. scrofa domestica SCD* gene based on the numbering of the sequence defined by accession number AY487830 in the NCBI database, wherein the presence in the two alleles of the *SCD* gene of nucleotide T at position 2228 and/or of nucleotide A at position 15109 is indicative of a high degree of fatty acid unsaturation with respect to the reference value.

### Methods of selecting pigs with high monounsaturated fatty acid content in muscular and subcutaneous fat

The identification of polymorphisms that are significantly associated with a high degree of unsaturation in intramuscular and subcutaneous fat allows the development of methods for selecting pigs having said trait by means of crossing pigs with said polymorphisms and selecting those pigs from the offspring that have inherited the beneficial polymorphism. Thus in another aspect, the disclosure relates to a method for selecting pigs with a high degree of fatty acid unsaturation in intramuscular and/or subcutaneous fat which comprises:
(i) crossing a pig having an A at position 2281 of the *SCD* gene, a C at position 2228 and/or an A at position 15109 in at least one allele of the *SCD* gene based on the numbering of the sequence defined by accession number AY487830 in the NCBI database with a pig of the opposite sex; and
(ii) selecting those individuals from the offspring having at least one of the polymorphisms indicated in step (i).

In a first step, the method of selecting and breeding pigs with a high monounsaturated fatty acid content comprises crossing a pig having an A at position 2281 of the *SCD* gene, a C at position 2228 and/or an A at position 15109 in at least one allele of the *SCD* gene based on the numbering of the sequence defined by accession number AY487830 in the NCBI database with a pig of the opposite sex.

The term "crossing" herein refers to both methods based on spontaneous mating and to artificial insemination methods using methods known in the art.

In a preferred disclosure, the pig of the opposite sex used in step (i) has an A at position 2281 of the *SCD* gene, a T at position 2228 and/or an A at position 15109 in at least one allele of the *SCD* gene based on the numbering of the sequence defined by accession number AY487830 in the NCBI database. The person skilled in the art will understand that when the two parents have polymorphisms favorable for a high degree of unsaturation of muscular or subcutaneous fat, step (i) can be carried out by crossing pigs having the same polymorphism, such that offspring that is homozygous for said favorable polymorphism can be obtained. Alternatively, step (i) can be carried out by crossing pigs having different polymorphisms favorable for a high degree of unsaturation of muscular or subcutaneous fat, such that the offspring incorporates the different polymorphisms. Alternatively, step (i) can be carried out by crossing pigs having the same polymorphisms, such that offspring homozygous for all the polymorphisms can be obtained in a single crossing and selection cycle.

In a second step, the method of selecting and breeding pigs with a high monounsaturated fatty acid content according to the disclosure comprises selecting those individuals from the offspring having at least one of the polymorphisms indicated in step (i). The selection is preferably carried out by means of genotyping of the members of the offspring, preferably using one of the methods described above in the context of genotyping methods. The selection can also be a "virtual" selection, such that the genotype of an animal is recorded in a portable database or computer. Here, the animals could be selected based on their known genotype without a need for physical separation. This would allow selecting the animals having the desired phenotype, where physical separation is not necessary.

In a preferred disclosure, genotyping is carried out by means of hybridization to allele-specific probes capable of detecting the nucleotide at position 2281 of the *S. scrofa domestica SCD* gene based on the numbering of the sequence defined by accession number AY487830 in the NCBI database in the *Sus scrofa domestica SCD* gene or in a polymorphic variant at position 2281 of said sequence. In a more preferred disclosure, the allele-specific probe comprises the sequence of SEQ ID NO:16 and allows detecting variants of the *SCD* gene, wherein the nucleotide occupying position 2281 of the *SCD* gene is a G nucleotide. In another preferred disclosure, the allele-specific probe comprises the sequence of SEQ ID NO:17 and allows detecting variants of the *SCD* gene, wherein the nucleotide occupying position 2281 of the *SCD* gene is an A nucleotide. The probes are preferably labeled at one of their ends with a fluorescent compound. In an even more preferred disclosure, the fluorescent compound is VIC or 5-carboxyfluorescein (5-FAM).

The selection of step (ii) allows identifying pigs comprising an A at position 2281 of the *SCD* gene, a T at position 2228 and/or an A at position 15109 in at least one allele of the *SCD* gene based on the numbering of the sequence defined by accession number AY487830. In a preferred disclosure, when both parents have the favorable alleles associated with a high degree of unsaturation of muscular or subcutaneous fat, the segregation of the alleles according to the laws of Mendelian genetics allows some members of the offspring to have the favorable polymorphism in both alleles. Thus in another preferred disclosure, step (ii) comprises the selection of those pigs comprising an A at position 2281 of the *SCD* gene, a T at position 2228 and/or an A at position 15109 in both alleles of the *SCD* gene based on the numbering of the sequence defined by accession number AY487830.

In a preferred disclosure, the method of selecting pigs comprises backcrossing a pig selected in step (ii) with a pig of the opposite sex. The pig with which the individuals selected in step (ii) are backcrossed are preferably pigs comprising one or more of the different polymorphisms which are markers of a high degree of unsaturation of muscular or subcutaneous fat and, in particular, an A at position 2281 of the *SCD* gene, a T at position 2228 and/or an A at position 15109 based on the numbering of the sequence defined by accession number AY487830 in at least one of the alleles of the *SCD* gene. In a preferred disclosure, the pigs that are used for backcrossing with the pigs selected in step (i) belong to the same litter.

The invention is described by means of the following examples which must be considered as merely illustrative and non-limiting of the scope of the invention. The following methods are common to all the examples.

### EXAMPLES

### METHODS

### 1. Study population

For the study of genetic variability of the porcine *SCD* gene, 780 base pairs of the promoter region and the entire non-coding 3' region (3'UTR) were sequenced in 12 pigs from the Duroc breed from the University of Lleida sample bank. There was a DNA sample and record of the content of 11 fatty acids in at least one muscle with respect to all the Duroc pigs analyzed (335 subjects). The 12 pigs chosen as the sample population of this study were chosen such that 6 of them had the highest oleic acid values in the semimembranosus and gluteus medius muscles of all those recorded (73.7 and 101.7 mg/g dry matter), and the 6 remaining pigs had the lowest values of those recorded for said acid (49.3 and 12.2 mg/g dry matter).

To sequence the *SCD* gene, by means of PCR six DNA fragments of between 780 and 1000 base pairs were amplified from the primers detailed in Table 2.

**Table 2: Probes (primers) used for the amplification by means of PCR of six fragments of the porcine SCD gene.**

| PROBE | 5'-3' SEQUENCE | SEQ ID NO: | AMPLICON SIZE (base pairs, bp) |
|---|---|---|---|
| Promoter-F | ACTTCCCTAGTGCCCATCCT | 2 | 890 |
| Promoter-R | GATCACTTTCCCAGGGATGA | 3 | |
| | | | |
| 3UTR_F1 | AAGTATCCAAGGGCTGCCATC | 4 | 866 |
| 3UTR_R1 | CAATTCCGGAAAGAACCTCA | 5 | |
| 3UTR_F2 | TGGGGAAGAAGTCTTTCTTGT | 6 | 990 |
| 3UTR_R2 | GGTTCAGTGACCCTGAGCAT | 7 | |
| 3UTR_F3 | TTTCCTGCCGGTTCTATCTC | 8 | 945 |
| 3UTR_R3 | GAGTAGGTGCTTGGGTCTGG | 9 | |
| 3UTR_F4 | ATGGAGGATAAAGGGGTTGG | 10 | 648 |
| 3UTR_R4 | ACTTGCCCAGGGTCACATAG | 11 | |
| 3UTR_F5 | GTCAAGGTTACACGGGTGGT | 12 | 742 |
| 3UTR_R5 | CAGGACATAGGGTGGCAGAT | 13 | |

### 2. Genotyping SNP3 mutation in the porcine SCD gene promoter

The polymorphism of the invention SNP3 was detected in 635 castrated pigs from the Duroc breed from the University of Lleida sample bank for which there was data available concerning IMF content and composition. The genotyped pigs were bred in 7 different groups. Genotyping was performed by means of an allelic discrimination protocol in a real-time PCR apparatus. The primers and probes used are indicated in Table 3 together with the final concentrations used.

**Table 3: Probes used in the genotyping of SNP3 of the porcine SCD gene promoter.**

| PROBE | 5'-3' SEQUENCE | SEQ ID NO: | FINAL CONCENTRATION |
|---|---|---|---|
| Forward primer | TGCCAGCTCTAGCCTTTAAATACC | 14 | 900 nM |
| Reverse primer | CACGTTGGGTCGGTGTGTCT | 15 | 900 nM |
| G allele probe | VIC-ACCCGCGCACAGCA-NFQ | 16 | 200 nM |
| A allele probe | FAM-AGACCCACGCACAGCA-NFQ | 17 | 200 nM |

The PCR reaction included the four primers, at the concentrations indicated in Table 3, 1x Maxima SYBR Green/ROX qPCR Master Mix (Fermentas), and 10 ng of genomic DNA. The genotype was assigned according to the accumulation of green fluorescence (corresponding to the VIC fluorochrome of the probe hybridizing with the G allele) and/or blue fluorescence (FAM, A allele) during the PCR cycles. Every genotyped pig was classified as GG, AG or AA genotype.

### 3. Determination of fat content and composition

All the muscle or fat samples were identified, lyophilized and ground. The C14:0, C16:0, C16:1 n-7, C18:0, C18:1 n-9, C18:2 n-6, C18:3 n-3, C20:0, C20:2 n-6, C20:4 n-6 and C20:1 n-9 fatty acids content was determined in duplicate from an aliquot of each of said samples by means of quantitative gas chromatography as described by Bosch et al. (Meat Science, 2009, 82: 432-437). The fatty acid methyl esters were obtained by direct transesterification from a 20% solution of boron trifluoride in methanol and were determined by gas chromatography by means of an SP2330 capillary column (30 mx 0.25 mm, Supelco, Bellefonte, PA) and a flame ionization detector using helium as the carrier gas at 1 mL/min. The content of each fatty acid was quantified by normalizing the area after adding to each sample 1,2,3-tripentadecanoylglycerol as the internal standard. The IMF percentage in a muscle was calculated from the ratio of the sum of the content of all the fatty acids determined, expressed in their triglyceride equivalent, with respect to the sample dry matter content. The percentage of each fatty acid with respect to the total fatty acids was calculated as the ratio of the content of that fatty acid in particular with respect to the total fatty acids. The MUFA percentage was calculated as the ratio of the sum of MUFAs (C16:1 n-7, C18:1 n-9 and C20:1 n-9) with respect to the total fatty acid content. The percentage of saturated fatty acids was calculated as the ratio of the sum of the saturated fatty acids (C14:0, C16:0, C18:0 and C20:0) with respect to the total fatty acid content.

The degree of unsaturation DU was calculated in a specific manner as the ratio of the C18:1 content with respect to the C18:0 content.

### EXAMPLE 1: Association between the genotype of SNP3 of the SCD gene and the DU of meat and subcutaneous fat of the pig.

The means of the three possible genotypes of SNP3 (GG, GA, AA) were compared in 7 different groups for the IMF content and DU in the gluteus medius muscle of the ham by means of a linear model that included the genotype as the sole factor. Table 4 shows the IMF and DU means by genotype, as well as the p value associated with the corresponding analysis of variance.

**Table 4: Effect of the genotype of SNP3 on the IMF content and DU in the gluteus medius muscle.**

| | | | | IMF | | | | DU (C18:1/C18:0) | | | |
|---|---|---|---|---|---|---|---|---|---|---|---|
| | | | | Genotype | | | | Genotype | | | |
| Group | Year | N | f(A) | AA | AG | GG | p | AA | AG | GG | p |
| 1 | 2006 | 109 | 0.44 | 14.50 | 14.64 | 14.97 | 0.91 | 5.93 | 5.90 | 5.37 | <0.01 |
| 2 | 2007 | 100 | 0.46 | 15.89 | 15.76 | 16.70 | 0.74 | 4.92 | 4.54 | 4.29 | <0.01 |
| 3 | 2008 | 66 | 0.44 | 16.52 | 15.15 | 16.31 | 0.40 | 5.96 | 5.12 | 4.54 | <0.01 |
| 4 | 2008 | 72 | 0.38 | 13.58 | 11.61 | 12.26 | 0.21 | 4.35 | 3.77 | 3.50 | <0.01 |
| 5 | 2010 | 85 | 0.39 | 14.76 | 15.27 | 13.84 | 0.54 | 4.35 | 4.34 | 3.92 | <0.05 |
| 6 | 2010 | 96 | 0.49 | 13.63 | 14.41 | 15.51 | 0.41 | 4.34 | 4.04 | 3.71 | <0.01 |
| 7 | 2011 | 86 | 0.48 | 18.78 | 17.66 | 16.91 | 0.41 | 3.98 | 3.82 | 3.73 | <0.05 |
| Total | | 614 | 0.44 | 15.27 | 14.92 | 15.18 | 0.72 | 4.80 | 4.51 | 4.15 | <0.01 |

It is consistently observed in all the groups that the A allele does not affect the IMF content but it does improve the DU. The effect of the A allele on all the groups overall (Total) was estimated by means of a linear model that included the group in addition to the genotype.

Similar analyses performed on the association between the genotype of SNP3 and IMF and DU in the longissimus dorsi and semimembranosus muscles confirmed the same effect, i.e., the A allele of said SNP does not increase the IMF content but it does improve the DU.

Similar analyses performed on the association between the genotype of SNP3 and the thickness of dorsal fat confirmed the same effect, i.e., the A allele of said SNP does not increase the thickness of dorsal fat but it does improve the DU.

### EXAMPLE 2: SNP3 has a mode of additive gene action

The mode of gene action of SNP3 on DU was verified in each group by means of a model with an additive covariate (a) and a dominant covariate (d), in which variate a has values 1, 0, and -1, and variate d has values 0, 1 and 0, according to genotype AA, AG and GG, respectively. Table 5 shows the values of a and d for DU, as well as the p value associated with each of them.

**Table 5: Additive effect (a) and dominant effect (d) of the genotype of SNP3 of the porcine SCD gene on the DU in the gluteus medius muscle.**

| DU | | | | |
|---|---|---|---|---|
| Group | a | p-value | d | p-value |
| 1 | 0.28 | <0.01 | 0.25 | 0.06 |
| 2 | 0.31 | <0.01 | -0.07 | 0.52 |
| 3 | 0.71 | <0.01 | -0.13 | 0.56 |
| 4 | 0.43 | <0.01 | -0.15 | 0.07 |
| 5 | 0.22 | <0.05 | 0.21 | 0.16 |
| 6 | 0.32 | <0.01 | 0.02 | 0.81 |
| 7 | 0.13 | <0.01 | -0.04 | 0.56 |
| total | 0.32 | <0.01 | 0.03 | 0.50 |

It is consistently observed in all the groups that the mode of action of SNP is strictly additive such that the DU increases between 0.13 and 0.71, according to the group, for each A allele present in the genotype. The mean effect of the A allele on all the groups overall (total) was estimated by means of the same model but adding the group effect. On average, each A allele increases 0.32 DU points.

### EXAMPLE 3: Association between the genotype of SNP3 of the porcine SCD gene and the percentage of MUFA and C18:1 in the fat of the pig and with other commercial traits

The effect of the genotype of SNP3 on the percentage of MUFA and C18:1 in muscle and subcutaneous fat was compared as these are the two DU-related traits having the greatest commercial effect on the quality of the meat and fat of the pig. They were compared by means of a linear model that included the genotype and the group with the IMF covariate. Table 6 shows the means of the preceding traits according to the genotype of SNP3 and the additive and dominant effects thereof, as well as the corresponding associated p values.

**Table 6: Effect of the genotype of SNP3 and additive (a) and dominant (d) effects of the genotype of SNP3 of porcine SCD gene on productive traits and MUFA and C18:1 content in meat and subcutaneous fat.**

| | | Genotype | | | | Additive and dominant value | | | |
|---|---|---|---|---|---|---|---|---|---|
| Item | n | AA | AG | GG | p | a | p | d | p |
| Age (days) | 617 | 209.1 | 209.0 | 209.2 | 0.90 | -0.01 | 0.98 | -0.17 | -0.65 |
| Carcass weight (kg) | 614 | 93.42 | 94.31 | 93.60 | 0.55 | -0.09 | 0.86 | 0.080 | 0.27 |
| Fat thickness (mm) | 582 | 22.24 | 22.49 | 22.49 | 0.80 | -0.12 | 0.56 | 0.13 | 0.67 |
| Lean meat (%) | 582 | 44.39 | 43.87 | 43.95 | 0.59 | 0.22 | 0.44 | -0.30 | 0.44 |
| Ham weight (kg) | 611 | 12.02 | 12.19 | 12.09 | 0.35 | -0.03 | 0.60 | 0.13 | 0.16 |
| Ham (gluteus medius m.) | | | | | | | | | |
| IMF (%) | 615 | 15.29 | 14.95 | 15.20 | 0.73 | 0.04 | 0.87 | -0.29 | 0.43 |
| MUFA (%) | 615 | 15.42 | 50.45 | 49.48 | <0.01 | 0.97 | <0.01 | 0.01 | 0.97 |
| C18:1 (%) | 615 | 46.61 | 46.02 | 45.28 | <0.01 | 0.66 | <0.01 | 0.08 | 0.61 |
| Loin (longissimus dorsi m.) | | | | | | | | | |
| IMF (%) | 236 | 11.30 | 11.39 | 11.11 | 0.85 | 0.10 | 0.76 | 0.18 | 0.69 |
| MUFA (%) | 236 | 52.58 | 51.30 | 51.50 | <0.01 | 1.078 | <0.01 | -0.26 | 0.25 |
| C18:1 (%) | 236 | 56.61 | 46.58 | 46.12 | <0.01 | 0.77 | <0.01 | -0.30 | 0.16 |
| Subcutaneous fat | | | | | | | | | |
| MUFA (%) | 103 | 51.44 | 50.26 | 49.68 | 0.11 | 0.88 | 0.04 | -0.30 | 0.59 |
| C18:1 (%) | 103 | 47.81 | 46.69 | 46.26 | 0.13 | 0.78 | 0.05 | -0.34 | 0.52 |

It is verified that on average each A allele increases the percentage of MUFA (C18:1) in ham, loin and subcutaneous fat by 1% (0.75%), such that substituting a pig or meat product having the GG genotype with one having the AA genotype improves the percentage of MUFA and C18:1 by 2% and 1.5%, respectively.

Table 6 also shows that the A allele of SNP3 does not affect carcass or ham weight (values of high commercial importance), nor does it affect the percentage of carcass lean meat or the IMF content.

## Claims

1. A method for genotyping a subject which comprises detecting in a sample from said subject the nucleotide occupying position 2281 in the *S. scrofa domestica SCD* gene based on the numbering of the sequence defined by accession number AY487830 in the NCBI database (SEQ ID NO:1), and wherein said subject is a pig.

2. The method according to claim 1 which additionally comprises detecting in said sample the nucleotide occupying position 2228 of said gene and/or the nucleotide occupying position 15109 in the *S. scrofa domestica SCD* gene based on the numbering of the sequence defined by accession number AY487830 in the NCBI database.

3. The method according to claim 1 or 2, wherein the detection of the nucleotide in the polymorphic position or positions said SNPs comprises hybridization to at least one probe specific for said SNPs.

4. The method according to claim 3, wherein at least one of said specific probes is a probe capable of detecting the nucleotide at position 2281 of the *S. scrofa domestica* SCD gene based on the numbering of the sequence defined by accession number AY487830 in the NCBI database in the *Sus scrofa domestica* SCD gene or a polymorphic variant at position 2281 of said sequence.

5. A method for predicting the degree of fatty acid unsaturation in a subject or a meat derivative thereof, which comprises detecting in a sample from said subject or from said meat derivative the nucleotide in at least one polymorphic position in the *Sus scrofa domestica SCD* gene, wherein said polymorphic position is position 2281 based on the numbering of the sequence defined by accession number AY487830 in the NCBI database (SEQ ID NO:1), and wherein the presence of nucleotide A at position 2281 in at least one allele of the *SCD* gene is indicative of a high degree of fatty acid unsaturation with respect to the reference value, and wherein said subject is a pig.

6. The method according to claim 5, wherein the presence of nucleotide A at position 2281 in the two alleles of the *SCD* gene is associated with a high degree of fatty acid unsaturation with respect to the reference value.

7. The method according to any of claims 5 or 6, which additionally comprises detecting in said sample the nucleotide occupying position 2228 of said gene and/or the nucleotide occupying position 15109 in the *S. scrofa domestica SCD* gene based on the numbering of the sequence defined by accession number AY487830 in the NCBI database.

8. The method according to claim 5, wherein the detection of nucleotide in the polymorphic position or positions said SNPs comprises hybridization to at least one probe specific for said SNPs.

9. The method according to claim 8, wherein at least one of said specific probes is a probe capable of detecting the nucleotide at position 2281 of the *S. scrofa domestica* SCD gene based on the numbering of the sequence defined by accession number AY487830 in the NCBI database in the *Sus scrofa domestica* SCD gene or a polymorphic variant at position 2281 of said sequence.

10. The method according to any of claims 1 to 9, wherein the sample is selected from the group consisting of blood, hair, epidermis, saliva, sperm, subcutaneous fat, muscle and of meat derivatives of said subject.

11. The method according to claim 10, wherein the muscle is selected from the gluteus medius muscle of the ham, the longissimus dorsi muscle and the semimembranosus muscle.

12. The method according to claim 11, wherein the meat derivative is ham or loin.

13. The method according to any of claims 5 to 12, wherein the degree of fatty acid unsaturation in intramuscular fat is determined.

14. The method according to claim 5, wherein the pig is from the Duroc breed.

## Patentansprüche

1. Verfahren zur Genotypisierung eines Subjektes, umfassend das Detektieren des Nukleotids an Position 2281 in dem SCD-Gen von *S. scrofa domestica,* basierend auf der Nummerierung der Sequenz gemäß Zugangsnummer AY487830 in der NCBI-Datenbank (SEQ ID NO: 1), in einer Probe von dem Subjekt, und wobei das Subjekt ein Schwein ist.

2. Verfahren nach Anspruch 1, ferner umfassend das Detektieren des Nukleotids an Position 2228 des Gens und/oder des Nukleotids an Position 15109 in dem SCD-Gen von *S. scrofa domestica,* basierend auf der Nummerierung der Sequenz gemäß Zugangsnummer AY487830 in der NCBI-Datenbank, in der Probe.

3. Verfahren nach Anspruch 1 oder 2, wobei das Detektieren des Nukleotids in der polymorphen Position oder in den polymorphen Positionen in den SNPs das Hybridisieren mit mindestens einer Sonde umfasst, die für die SNPs spezifisch ist.

4. Verfahren nach Anspruch 3, wobei mindestens einer der spezifischen Sonden eine Sonde ist, die in der Lage ist, das Nukleotid an Position 2281 des SCD-Gens von *S. scrofa domestica* zu detektieren, basierend auf der Nummerierung der Sequenz gemäß Zugangsnummer AY487830 in der NCBI-Datenbank in dem SCD-Gen von *Sus scrofa domestica,* oder eine polymorphe Variante an Position 2281 der Sequenz.

5. Verfahren zur Vorhersage des Grades an Fettsäure-Unsättigung in einem Subjekt oder einem Fleischderivat hiervon, umfassend das Detektieren des Nukleotids an mindestens einer polymorphen Position in dem SCD-Gen von *Sus scrofa domestica* in einer Probe von dem Subjekt oder von dem Fleischderivat, wobei die polymorphe Position die Position 2281 ist, basierend auf der Nummerierung der Sequenz gemäß Zugangsnummer AY487830 in der NCBI-Datenbank (SEQ ID NO: 1), und wobei das Vorhandensein des Nukleotids A an Position 2281 in mindestens einem Allel des SCD-Gens einen hohen Grad an Fettsäure-Unsättigung im Vergleich zum Referenzwert indiziert, und wobei das Subjekt ein Schwein ist.

6. Verfahren nach Anspruch 5, wobei das Vorhandensein des Nukleotids A an Position 2281 in den zwei Allelen des SCD-Gens mit einem hohen Grad an Fettsäure-Unsättigung im Vergleich zum Referenzwert assoziiert ist.

7. Verfahren nach einem der Ansprüche 5 oder 6, ferner umfassend das Detektieren des Nukleotids an Position 2228 des Gens und/oder des Nukleotids an Position 15109 in dem SCD-Gen von *S. scrofa domestica,* basierend auf der Nummerierung der Sequenz gemäß Zugangsnummer AY487830 in der NCBI-Datenbank, in der Probe.

8. Verfahren nach Anspruch 5, wobei das Detektieren des Nukleotids an der polymorphen Position oder an den polymorphen Positionen in den SNPs das Hybridisieren mit mindestens einer Sonde umfasst, die für die SNPs spezifisch ist.

9. Verfahren nach Anspruch 8, wobei mindestens eine der spezifischen Sonden eine Sonde ist, die in der Lage ist, das Nukleotid an Position 2281 des SCD-Gens von *S. scrofa domestica* zu detektieren, basierend auf der Nummerierung der Sequenz gemäß Zugangsnummer AY487830 in der NCBI-Datenbank in dem SCD-Gen von *Sus scrofa domestica,* oder eine polymorphe Variante an Position 2281 der Sequenz.

10. Verfahren nach einem der Ansprüche 1 bis 9, wobei die Probe ausgewählt ist aus der Gruppe bestehend aus Blut, Haaren, Epidermis, Speichel, Sperma, subkutanem Fett, Muskeln und aus Fleischderivaten von dem Subjekt.

11. Verfahren nach Anspruch 10, wobei der Muskel ausgewählt ist aus dem Gluteus-medius-Muskel des Schinkens, dem Longissimus-dorsi-Muskel und dem Semimembranosus-Muskel.

12. Verfahren nach Anspruch 11, wobei das Fleischderivat Schinken oder Lende ist.

13. Verfahren nach einem der Ansprüche 5 bis 12, wobei der Grad an Fettsäure-Unsättigung in intramuskulärem Fett bestimmt wird.

14. Verfahren nach Anspruch 5, wobei das Schwein von der Rasse Duroc ist.

## Revendications

1. Un procédé de génotypage d'un sujet qui comprend le fait de détecter dans un échantillon provenant dudit sujet le nucléotide occupant la position 2281 dans le gène *S. scrofa domestica SCD* sur la base de la numérotation de la séquence définie par le numéro d'accès AY487830 dans la base de données NCBI (SEQ ID NO : 1), et dans lequel ledit sujet est un porc.

2. Le procédé selon la revendication 1 qui comprend en outre le fait de détecter dans ledit échantillon le nucléotide occupant la position 2228 dudit gène et/ou le nucléotide occupant la position 15109 dans le gène *S. scrofa domestica SCD* basé sur la numérotation de la séquence définie par le numéro d'accès AY487830 dans la base de données NCBI.

3. Le procédé selon la revendication 1 ou la revendication 2, dans lequel le fait de détecter le nucléotide dans la ou les positions polymorphes desdits SNPs comprend une hybridation pour au moins ou une sonde spécifique pour lesdits SNPs.

4. Le procédé selon la revendication 3, dans lequel au moins l'une desdites sondes spécifiques est une sonde apte à détecter le nucléotide en position 2281 du gène *S. scrofa domestica SCD* basé sur la numérotation de la séquence définie par le numéro d'accès AY487830 dans la base de données NCBI dans le gène *Sus scrofa domestica SCD* ou un variant polymorphe en position 2281 de ladite séquence.

5. Un procédé pour prédire le degré d'insaturation en acide gras chez un sujet ou un dérivé de viande de celui-ci, qui comprend le fait de détecter dans un échantillon dudit sujet ou dudit dérivé de viande le nucléotide dans au moins une position polymorphe du gène *Sus scrofa domestica SCD,* ladite position polymorphe étant la position 2281 basée sur la numérotation de la séquence définie par le numéro d'accès AY487830 dans la base de données NCBI (SEQ ID NO : 1),
et dans laquelle la présence du nucléotide A en position 2281 dans au moins un allèle du gène *SCD* est indicative d'un degré élevé d'insaturation en acides gras par rapport à la valeur de référence, et dans lequel ledit sujet est un porc.

6. Le procédé selon la revendication 5, dans lequel la présence du nucléotide A en position 2281 dans les deux allèles du gène *SCD* est associée à un degré élevé d'insaturation en acides gras par rapport à la valeur de référence.

7. Le procédé selon l'une quelconque des revendications 5 ou 6, qui comprend en outre le fait de détecter dans ledit échantillon le nucléotide occupant la position 2228 dudit gène et/ou le nucléotide occupant la position 15109 dans le gène *S. scrofa domestica SCD* basé sur la numérotation de la séquence définie par le numéro d'accès AY487830 dans la base de données NCBI.

8. Le procédé selon la revendication 5, dans lequel la détection du nucléotide dans la ou les positions polymorphes desdits SNPs comprend une hybridation pour au moins une sonde spécifique pour lesdits SNPs.

9. Le procédé selon la revendication 8, dans lequel au moins l'une desdites sondes spécifiques est une sonde apte à détecter le nucléotide en position 2281 du gène *S. scrofa domestica SCD* basé sur la numérotation de la séquence définie par le numéro d'accès AY487830 dans la base de données NCBI dans le gène *Sus scrofa domestica SCD* ou un variant polymorphe en position 2281 de ladite séquence.

10. Le procédé selon l'une quelconque des revendications 1 à 9, dans lequel l'échantillon est choisi dans le groupe constitué par le sang, les poils, l'épiderme, la salive, le sperme, la graisse sous-cutanée, le muscle et les dérivés de viande dudit sujet.

11. Le procédé selon la revendication 10, dans lequel le muscle est choisi parmi le muscle moyen fessier du jambon, le muscle *longissimus dorsi* et le muscle semi-membraneux.

12. Le procédé selon la revendication 11, dans lequel le dérivé de viande est du jambon ou de la longe.

13. Le procédé selon l'une quelconque des revendications 5 à 12, dans lequel le degré d'insaturation en acides gras dans la graisse intramusculaire est déterminé.

14. Le procédé selon la revendication 5, dans lequel le porc est de la race Duroc.


### SEQUENCE LISTING

<110> UNIVERSITAT DE LLEIDA
<120> POLYMORPHISMS ASSOCIATED WITH THE DEGREE OF UNSATURATION OF INTRAMUSCULAR FAT IN PIGS
<130> P8396PC00
<150> ES P201231507
   <151> 2012-09-28
<160> 17
<170> PatentIn version 3.5
<210> 1
   <211> 20985
   <212> DNA
   <213> Sus scrofa
<400> 1 
   tctagaccct ttgaatttcc acttaaattt tagaatctgc ttgtctattt cctcaaagaa 60
   atctgttgga tttctatagt gattaaatta atctatagat caacactgag tctgtccatt 120
   tatgaacaca gaatataaca aattcttttt ttctctctat ttaagagttt taaagttttt 180
   ctcagcattg ttttgatact ttcagtgtag ctatcctata aacatttaga tttattcttt 240
   gtgacatttt tgatgttctt ttaatttttg tttttttagg gccgcacctg tggcacatga 300
   agttcctggg ccaggggtcg aatcagagcc aaagctacca gcctatacca ccaccatagc 360
   aacgccaggt ctggaccgca tctgtgatgt acgccggagc tcgtgacaat gccagatcct 420
   taacccactg agcaaggcca gcatgtaacc cacaacctcg tggacactag tcgagttctt 480
   aacccgctga gccacaagga gaactccatt tttgatgcta ttttaaacag tatattttaa 540
   atttcattct gcaattgttt tttaatagaa tataggacag aataattttt ttatactggt 600
   ttctatcttg acctttctaa attcacttac caattttgac actttttggg tggtagattc 660
   tatacatttt tcatacacac agttaggtct tctgcaaata ataacagttt ctttcttctt 720
   ttcctatctt tatatctttc atttcttttt tcctgtccat catgcaacct aggatttcta 780
   gcacaatgtt gactagaaat ggtagacgat tatatgcttg cctgttctct taaggggaaa 840
   tttttttttt ttttttttgg tctttttaag cctgcaccca tggctaggaa ctgtagctgc 900
   tggcctatgc cacagccaca gcaacactgg atccgaactg tatctgtgac ctacaccaca 960
   gctcatggct atgccagatc cttaacccac tgagcgaggc cagggatcga accctcgacc 1020
   tcacggttcc tagtcggatt cgtctccgct gcgccacaac gggaactccc acgactaatg 1080
   attaagtaaa atgattcttg tggactgact tgcctaataa ttctatagct gatagctatt 1140
   tttgtcccac ttacaagtcc ttttgcctgc ttctggatga aggcacacgc tgcgcttcct 1200
   cccttttctc atgaaagcca ttccaaaatt acaactcgac aatctgaaga atttccaaaa 1260
   catcccgcac acgttcgggg tggggggggg gcgtgggcac aaggacggga tcgctcactg 1320
   gtgcctggcg gcgaacgtcc cctgctgggc ttgcctagta gctgaacgcc gtgcgcgcgt 1380
   gcaggtccac acatctatct atgcatctac gtgtgtgtac acggctgccg gcctggagac 1440
   cggcgagttc agcagtacgc atctatcgcc ccgccgccag tgggcggcgc agagccattg 1500
   ttccgccgcc ggccgaccct cagcccctgc ggcacgcgcg ttcgtcacgg agggaggtgg 1560
   ggcttcgccg ggcccccggc ccccaacccg gggtttgaag gcacgtctcc ccctcccacg 1620
   cgtctccgcg agctagaagg cgggagtgga gttgtgcgta gcggtcgccc gcgcggcttc 1680
   acccacactt tccccggaaa cttccctagt gcccatcctt tcgcgaagtg ccccagggcc 1740
   agtcctgggc tagcacccac ctcccacccc cctactgagt cctcttcctc ttccagggaa 1800
   gacgctctcg agcgcctccg aggaggcagg acgccgggca gaagcccagc ggccggtgga 1860
   agagaagctg aggaggagaa agggagggga gggggagtga ggagctcgcg gcagagggaa 1920
   cagcagattg cgccgagcca atggcaacgg caggacgagg tggcaccaaa ttcccttcgg 1980
   ccaatgacgc gccagagtct acagaagccc attagcattt ccccaggggc aggggcagag 2040
   gcaggggctg cggagaccga gccgcggagt gtctgcagca tccagttttt gcgtctccgg 2100
   cccccagcaa gccccggctc tctgtctcct cccctctccc gcccatgcgg ttctcccacg 2160
   gtgagccaac tctgcgcact ttgccccttc ttggcagcga ataaaagggg tcagaggaaa 2220
   tacaggatac ggtcgcccac tgccagctct agcctttaaa tacccagcct ggggagaccc 2280
   acgcacagca ggtcgggtgc agacaccgac ccaacgtgca gcaaagggtt ccgagcgcag 2340
   catcgctgat ctccgcgcaa gagccggctc cagcactagt ctacgctcag tgcggtctgc 2400
   cacgaactcc tccctgaagt ctcatccctg ggaaagtgat cccaacgtcc gagagcccag 2460
   atgccggccc acttgctgca agaggaggtg agtttccaag taatggcccc cagaccgcgg 2520
   gctcgccagt gctggttggg cttctaggtg actcggtgta gaagagagtt gagatcgccg 2580
   aggacagcca cctttttcga gttgtatagt cttcagtttg ttgggaatgt ggattgtaat 2640
   ttgggaactt agttctccaa cttttgtttc ctaaagcttc aaagaaaaat ctggtggtac 2700
   ctgtaccctg atgtgtgtag gcatgcgggc tttcctttgt gtgtttgaag gggtgcgagt 2760
   gttttacccc tctatttccc cttcccctga ggtttcaccc tcctccccaa tttctccgca 2820
   cagttcctgg ggacttgcgt ctcttccatc taggatagac cttgccctca ggtaacctgg 2880
   gtattggggc caggtctgtg cctatacccc gcccttcccc cagctagacc agggagtgaa 2940
   gtggccccta gtgtctccac gtgtcctccc ctctctccca gctctgcact tccactctct 3000
   ttcagatctc tagctcctac acaaccacca ctaccatcac agcaccttcc tccagggtcc 3060
   tgcagaatgg agggggcaag ttggagaaga ctccccaata cgtggaagaa gacatccgcc 3120
   ctgaaatgaa agatgacatc tatgacccaa cctaccagga taaggagggc ccaaggccca 3180
   agcttgaata tgtttggaga aacatcatcc tcatgagtct gctacacttg ggagccctgt 3240
   atgggatcat attgatcccc acctgcaaga tatacacctt gctctggggt aagaggtccc 3300
   cttgtcctcc tgtctcagta tcccaggttc tctccactct tttaatacag tagcaactta 3360
   cacagagtag aaacaccagc ccctccaggc cgaggttttt ccaaccattt ctcttaggtg 3420
   ttgtggtggg ctgggaaggg agtctagggg gtttgggatg caggagtgtc gattctgggg 3480
   cctttaattt gggggggggg ttggagaggg agccctggga ccatgggcca caggctttga 3540
   agtgagtaga tctgtgccag ggagtcagct tttctgtttc aagtcatttt cttgactgct 3600
   tgtcccagcc ctgccagcct agaaagaatc aaggccctta cctggatgca tgggaacatg 3660
   gggctagact agtgtgactt tagagggcac atgctgggta tagtcacggg gtagcaagag 3720
   aaactactca ggaagagggt taagcagaag aggggggaag agagctgtgg tcatccatga 3780
   aattattttg ccctgatcct tagccttatc aggctcctta attacacaga cagaggaagg 3840
   cggctcctgc ataactcatg ttacatggat gtcttgctct gtgactccac gtgggtctcc 3900
   agggaactgc acatgccact caagaaaatg ttctaatgaa ataaggttaa agaaacgttt 3960
   cctgccccat tccccaccca tcctgctcca gcccctctcc cctgatgcca ggagtgcctc 4020
   cttgctgggg acagccaagc cagaaaagag actccggagc agccccggcc ctcaggctgc 4080
   tgcagtgagg gagacagtcc cccgtgcgtc cctgacctgc ccttagctca gctctttcag 4140
   gaatccatgg cttgggctga tagtggagcc ctgccccctc acacacatgc gctctgcagg 4200
   cattgccact cctgcctcga gtctgacttg ggaagaggag agcagggaac tctaaagatc 4260
   caggagctcc atgctgctac cctctgtgat gggtgtgggg ttcagaactc cctgttcaga 4320
   aagccacggt atggtttctg gagcagtgtc ggatgttggg tccttgctgg cagctatgct 4380
   cttggagggt ggttctacag aaggactggt gggccaagtt tcagcaaagt cctccaggac 4440
   ctctttgatc taaagagggt ggatctggtc tgcggagcct tgtcttggca gatcctcttg 4500
   gtgccacaga cagagaaatg catctgggta gattccctct ctcaattttc ccagacacaa 4560
   actggggaca acaggacttt ttttaagctt agaaaggtag tgctcctctt ttggcctccc 4620
   tgaagcatgt ggagttgccg gaccggggat cagacctaag cctcagctgc aggaatgccg 4680
   gatccttaac ccaccctacc aggccgggat tccacctgag tcccagcatt cccaaggtgc 4740
   cgctgatccc gttgtgctac agcggacatt ccaagaaagg tgctcctgga gttcctcgag 4800
   tgtcattgct ggggccagcc aggtgcagag gaaatcaatt catcctcaga ccaggaaggg 4860
   ctcttataat tgtaagggga gggggacttc tgctccccag cccctctgag aacccagcag 4920
   agcttgagtt gcagtgtgag gaatcaagag ccagggacat ggatgcccca gggaatctgt 4980
   gggctcccct cctgacccct tctcccccat agaggagtct tcacaagttt agtcacttcc 5040
   aagtccccct ccagcctcag gactccctgt cggcctcgtt aagttccgag tttggccagg 5100
   cagctgctgc tggctcttct cccttggctg ctactgcagg ggacaggatg ctgtccccaa 5160
   ccaaggctgt gcctgccgcc tttttggtgc cctgcctgta gatgtgtgct ctgcccttgg 5220
   acagaaacct gtcacacaga ctggatggtg acttttttac ttgtccctgg atcctgtggt 5280
   ggtggatggc ttagctgatg gaatgaccat tttggtatgg ccatatccga gctcctctgg 5340
   tggagttggg gggagggtca gagttcccga accttaagag ttcaggaact tggcttcatc 5400
   ttttcttgtc ttgtccctaa ggacagtgct gaattagcaa atggttgtga ttccagcatt 5460
   tgtcaggact gagcaatttt gagtgtctca gaagagccct gagggctgaa gcgatacctg 5520
   gcagcacctg gggcctctga ggcgaaacgt ctccaggctc ttccttaggg ctaaaactat 5580
   gaagttttgc ttaaccataa atctagactt tagtagagaa tgaatatcca actgacaggc 5640
   ttaggggtgc tggtagaaaa aggcaccaga gacttgggtg tttccccagg ttctctgtta 5700
   ggatcatatg tgcttccatt tttgtgcttt gcttttttag ggccacactt gcggcatatg 5760
   taagttccca ggctaggggt caaatgggag ctgcagttgc tggcctacac cacagccaca 5820
   gcaactccca cgtacaccac agttcatggc aatgctagat ctttaaccca ctgagtgagg 5880
   ccaggcatca cacccatgtc ctcatggata ctagtcaggt tcgtaattca ctgagccaca 5940
   gtcagaactg cctgcgtgtg ctttctgagt cagcttgcca gggtcttcgg ttgcatgtaa 6000
   ccaccccagt agcttcctgg gttctgagct acctctttcg tgctcaaaga cttttcttgg 6060
   cctttggtga ttggcgggct gcggctcaac tgttatttat ccaggtgcag atgcaggcag 6120
   atgctgcctc tgggaggata ttaccacaag gcgggggagg ccagaaaatc acctggaccc 6180
   ctaatgcagc cccgaggacc gtgggtggca cttccctgag acagactctg gctccctggc 6240
   tctcctccct gtcctgtagg aggaataggc tagggtagag gaaggtgggt gggacagggg 6300
   gcttgtttag gaaactggtg tctgtggctg agaacacaag acaatgtgtt acaatgtgtg 6360
   gtgtctccaa ctgcaactaa gttcagtggc cactgaggat ccattgtttc tagctgcttc 6420
   cctaggaatg aaacattgtc aacagtcact gctctcccaa ggcctcagcc tctgcctcct 6480
   agagctgctg aacagaggaa ggtgggtgtg cccagcaacc cgcctcccag ttagagactc 6540
   caacctgcac ccctagaacc tgcaggcccc caggaagatg aggtagacag cgctggggtg 6600
   ccccccacca cagctttgag cctgccctac cgttgcctag aattaaccct gacgggggta 6660
   tgagctgact gaggaaggtg gcaaaggaga gatgcaggag cgctgcgagt atcttgggcg 6720
   gggggggcat ggtaaacatc ctcgagccag ggctcaggag caccaagtac ttgttgacag 6780
   aacacctcat ttcaaaccat ggctccattt tttttttttt tttccttttg ctttttaggg 6840
   ccacaccttc ggcatatgga ggttcccagg ctaggggtct aatcagagct gtagccaccc 6900
   acctacacca cagccatagg aactcaggat ccgagccgca tctgcgacct attccatagc 6960
   tcactgcaac gccagatcct taactcactg agtgatccca gggatcgaac atgcaacctc 7020
   atggttacta gttggattag tttttgctgc gccacaacag gaactcctat ggctcccttt 7080
   cttactagcc atgtggcctt agataaacac ttaatctttc tgagtgtcta tttctttggc 7140
   ttaaacccca tgcaaggtat ccagtgaatc aaagaataca gaaatctgga gttcccctta 7200
   tgtctcagcc gtaacgaacc tgactagtat ccataaggac tcgggttgat ccctggaccc 7260
   gctcagtggg ttaaatatcc agcattcctg taagctgtgg tgtgggttgc agacacagct 7320
   ggggtctggc gtggctgtgg cgtacgttgg cggctgtggc tccaattcca cccctagcct 7380
   gggagcttcc atatgctggg gatgtggctc tagaaacaaa cacaaacaaa caaacaaaaa 7440
   tccttggcaa agcttaagac tgtgcagata tgacttaggt gcaaggcgag ttttccccat 7500
   tctgtgacca cgcagtctgc caaaagactg aaatggtttt gtaccagatg gtaaatagct 7560
   ctagttctga gccccccccc cgccccccgc tgccccccat gtcccctaga acccagagtc 7620
   cctcaaactg gtcagtgccc aggccatcct gtcttacata ttttaatact gcccttgttt 7680
   atgggtcttc agctctgcaa gagtactctg tggtccttaa aactgtggag aagcttagtg 7740
   aaggtcagag gagatgggac ctgggataat gtccttcatg acagagaatg acaagtggct 7800
   ggagggtgag agccctcttc tctgtcccgc cttcctgaac tttctgggac ctgtaagctt 7860
   attccagctg tgaggctaag aggataggag gcgatggtaa tttttttcca agtggtaaaa 7920
   ttgaagggga ctaagagatc taggtgtccg tggagttcag ggaaaagcag atcctcacct 7980
   gcagcctgaa ggacacctag acgctgacct gaggagaaca tcacgtgtaa gacttgcccc 8040
   gattactgac tccctgtcct gtcttccggc agcgtttgcc tactatctgc tgagtgctgt 8100
   gggtgtcacg gcaggagctc accgcctgtg gagtcaccga acttacaaag ctcgactgcc 8160
   cctgcgggtc ttcctgatca ttgccaacac gatggcattc caggtaagaa gccagctgtg 8220
   ctcagctctt tatcaaacgc aggcgatggg tgcctggaag ggagcagcac ctggacagct 8280
   acttcacttt cctctctgct tccagtcacc tcaagttcag gttcagtcct tgggtccata 8340
   acacctgcag tactttggag tgactggaaa agggaaatag gggataaaga ggaaatagcc 8400
   tctgtgtata ggatcgtttt cgagcctccg ttgtcacaca gtctggtcgt tgtggtccct 8460
   tcattagtgc acgaggcagg aactgaccct gatttcctag gcagccgttg gacaatacat 8520
   tcactatttt aggtgaacat ctcaggactc caccaaaagg gattaaagtt aatgtttcac 8580
   agattcatga accctattgt tttcaggtga gaagatcaaa cacttttata gtgttaacta 8640
   tacctcctgg ccctattctg agcacttgac aaggattaca ccattaagcc ctcccaacag 8700
   tcttaggagg ttggtactgt tgtgatcccc atttcacgga gggggaagct gtgtcactga 8760
   gggtgtaggt agaccatcat tatggtcctg tgttgagctg ccaagctgtc taagtcaaat 8820
   attgaggtct gctgaagccg gaagctcata gcagggaatt ggagaaaggg aactccgagt 8880
   tattgaacag cagcactggg caaaggctag catgttgtgt ggggcttagc ttggatcaag 8940
   gggcaaaaag aaggaaacca aatcggttcc tttcttcgtg gcagtgtctc acacagctgc 9000
   ataactgtct tctgccatag cacttaacgc cttgaaggcc cctgatgtgg tccctgttgg 9060
   ggcaaaagaa ggaagagata ttggcaccct ctctaagcga tagaaagtgt cccagaggag 9120
   cccagagacc atttggaaat gcggtcaaat catgattatg tgtctggcct tgttgagttg 9180
   tgggagctga atcttgccaa gggattaggc agcctgtgaa gaccctttgc tccagttctt 9240
   gctggaagaa ttgcaatttt acctcctagt gaaactcttc aagctctata caatccatct 9300
   ccattctgcc caccatataa cccgcaagaa gcagccagcc ctattcatgt ggtcctgaga 9360
   cttgaaggtg cttttccact taagcatcag tggccagtta gagaggaggc aactccctga 9420
   ctcctccttt ggagccctga ctcccaggta tttcgcgagc ttgggctgag cgcctcgggc 9480
   tcttgaagac acctagccat tggcattccc ctgagagggt gtctctcctc aggcctccat 9540
   tcctcttctc tgtccctcca gaatgacgtt tatgaatggg cccgagatca ccgtgcccac 9600
   cacaagtttt cagaaacaga tgctgatccc cacaattccc gacgtggctt tttcttctct 9660
   cacgtgggtt ggctgcttgt gcgcaaacac ccagccgtca aagagaaggg tggtttgctt 9720
   aacatgtctg acctaaaagc cgagaagctg gtgatgttcc agaggaggtg agtgctgtgg 9780
   tggtggagct gggcgcctgg tgtttgggga caccctctcc cctccctgtt ttttcttcta 9840
   ggacttgtcc acataagctg ggaaactgac agggtctgcc taccccaaat cataattcaa 9900
   aacccttctt ttatgggcca cagccataga ctgttgttga ttctttctct ctgagctaaa 9960
   aaaaaaacca gttaaggtca acaatgtgtg ttgacctgtt acagtcatgg atacaaagct 10020
   acatgttgcc atagtcctta ccttcaagaa gttcatagcc tgttggggat gggagcacta 10080
   agccaggtgt ccaaatacct atgacatggc aaaatatttt tctgtcccca ggaaaggcac 10140
   agaggggagg gagactgtgg agtggggaac attagagaag ggttttagga gaaggtagct 10200
   taggaatcca ctggtggata gagattttca caagtgttgg cggaggggga ggcagtgcaa 10260
   acagaaagaa tgagctaaag catgaagcat gttccagcaa tagagtccct ctgcttaaag 10320
   tacagggtgt ggtaggaaat aaagactaga gagaccgaga cctttcttgt ggggccttaa 10380
   gagcagacag ggtttaattc aactccagca ataggaaacc atggaagggt ttttgagccg 10440
   gaatgtggtc agaggtgtgc tctggagagg tcaccctgca gataggtgaa agaaaccatt 10500
   gggctgaaga ggtgagctgc agggccccag gtgaggaggt actgggcatc tgtgctgggg 10560
   aagtggtggc acggagagga ggcgaaatgt ggggacacac gtgatccaag agtagcagac 10620
   ttgactgctg aagaggtgca ggaaatcagc tccctcggaa tgtcagtagc ctgttttact 10680
   ttgggagaat gaagaatggg ccagggcctc cagagggaca gcctgcaggc atccgggaca 10740
   ctcggtgtgc cctgaggggg aaaagaagag aaaggatcag aaggcttgct ggctagcaag 10800
   agaggaaggc aagataccaa acacagattc tgcaaagagc actgacacca agagaggagc 10860
   aggcagaatg gaaagacctg ggcaggagaa ctcagttcag taatctgtaa tttgggccgc 10920
   ccctgttgtg gtacgattag gctgtggtca aagagagcaa tggatggaga gtcgggagac 10980
   aaggcattcc agcccttgct ccactggttc ctggtggtat gaccctagaa aagtcattta 11040
   gcccctcctt accttacctc atcaaaaaag tatgataata acactgcctt ctcaaaagac 11100
   tacccacccc atccctcttg ctctgtttcc tgccaccttg tgctcacagc gtagagggta 11160
   ggggacagct cagccccatg aaaggacaaa gtcagagtcc cctccatgaa tggggtgtgt 11220
   gatctctccc tgtaggtact acaaacccgg tatcctgttg atgtgcttca tcctgcccac 11280
   gatcgtgccc tggtattgct ggggtgaagc ttttccccaa agcctgttcg tcgccacctt 11340
   tcttcgttac gccatcgtgc tcaatgccac ctggctggta aacagtgctg cccacctata 11400
   cggatatcgc ccttatgaca agactattag cccccgggag aatatcctgg tttcattggg 11460
   agctgtgggt aagttagcag tccatagcaa gaccccatct agtgggctgc tggttaagag 11520
   tattagacta ggagccagaa aaaccagatt aacctgcttt atgacccctc tctgtttctc 11580
   tgctataaaa ctaaggtgca atagtagata ccccttgaga gttgggcagc aagtcttata 11640
   taaagagaat tgggctaaaa aataacaatg tctctgagtc caggttccaa aaagatcccc 11700
   aggtgctctg gatgaggaac cgctttggct tgtccttttt ccagtcatct ctgagctggc 11760
   acatggtctg gctaggggca gggactgcat gaatagaaca gtagggtctt ctgggtggcc 11820
   agtaagccta gtgttttata tttagacatg ctatacaaaa tgcagaacaa atccttaaca 11880
   tttcaacaag cttctctttt atcaccttgc tatattcaga gaggctgaac tcaaagtgta 11940
   tttcatgcct tattgtttgg tgatgccaac atgaaggacc tcaaagcaac tgttggtggc 12000
   ccttttatca ccgccgaccg ctctcatctc ttgtccttta ttcttctctt gtcctttatt 12060
   cttctctaac ccgtgctggg aacacccgct ccccttaaca tggcttggag aactcctgat 12120
   aaagcagtta atttgatcac aagtctctgc tcactgatct aatgctgact attcaagaat 12180
   atctttctaa aaaaaaaaaa agttatatct ttctctcatt ccagttgcaa tagccagaat 12240
   ctaagacaac agtgttctac catctgtctc ccttttattt tttcccctgc cactcttttc 12300
   aactaaacat agatcttgac taacgcctgg aagcagtttt ccttaagcag cgcctgtgac 12360
   ctcttccacg ccagtgatca gtgccactgg ttgtctggca tatctaggca acctccctgc 12420
   tccccacatg tgtgtacact gcggcctagc ttttgctttg ttctctttaa aacctttctt 12480
   tatgtgctca accctgagca tctttttttt tacaatctgt actaaagaca tttcatttgt 12540
   atcttttttt tttttttttt tagtgccgca cctgtgccat atggaagttc ccaggctagg 12600
   ggtccaatcc gagctacagc tgctggcctc caccacagcc acggcaatgc cagatccaag 12660
   caacctacac cacagctcat ggcaatgctg gatccttaac ccactgagca aggccaggga 12720
   ttgaacccac aacctcatgg ttcctagttg gatttgtttt tgctgagcca caaggggaac 12780
   tcctgtatct ctttttaact ttcatgtatc ttccccctcc tttcagggac cctctaccat 12840
   tgtgcatgta tctactctga accatgtttg aaatctaaca ggctattttc atagatttat 12900
   gaatgttgcg gaagtttctg gaatgagact ggtcctggat ttagaatgag ctcccccaac 12960
   cccagtctct ttctaaccat ccgttcaaag tagctgccaa catcttcctt tgtctcaatt 13020
   atcattcatc cctccatcct ggctgctctc ccccttatta cctcaccctt tgttctccca 13080
   tagctggctt ccctgtcatt ctaatctttt tcttcttgaa ttacctcccc cacagtcaca 13140
   tgctcatctc tccgctaaag ctgacctcct gccttgagcc tctcctggac tgtgtcttgc 13200
   tgccccagtt gcagagtctt gtccttctcc tctttttttc tcccttggca actagtgtgt 13260
   gtctgattaa agttttaaat ctgataagta tatctgataa gcctggctag tgcttatcaa 13320
   gatcctagct gcagaatttt atggaaattc gtacttttag ccttggggtt atcctaccct 13380
   cctactcagt tcacactaga taagctgatt atccacttgg tagagagtca gacagaggac 13440
   ctacctcgaa tgatgccctg gatggtgtca tttcctttgc agaatttcct tccttgcttg 13500
   ttattaatgt cattactact ctactaaggc tctttcatct tcaaagggct caacatgctc 13560
   ttttattagc tattcttgcc cagctctgcc tgggcgtcat aaggggtcag cattgtcata 13620
   gttgaatgct gttcggaaaa aagatcccgc caagggttgg agacctctgg ctctggagtt 13680
   tgcacagctc aactcatgac agacattatt ctgaatccag agtttctctg attttcttct 13740
   atggagcaaa gtgaactctt ctcagaagca tagaggtttt ttattctaat tctaagcctt 13800
   ttgattctgt ttccctcaga gagcttctgt cttatcagct tgacccccac accaggattc 13860
   aagctcaaag aattagaagc cagagcagtc ccctctgaac cagtggaaac tgaggaaatg 13920
   tgggcccctg gtctgggcct ctttctgact tttgactgtt ggtgcctgat tccgtggtac 13980
   cttctgccct tagagatggt atggtacagt ggggaaaggt ataccccctt gctcaccttt 14040
   ttcagaaaca cggcagcagt tctttgttct agactcttct ctcctcaccc taacacatgc 14100
   tcactcatac agagctttaa ggtctcagac caataactag tgaagctctg gaatttccca 14160
   atctaaaatt tatcccctgt gatagcagtt tctctccggt catcttatcc cctcagagga 14220
   actgtcagtc ctcttcccac agaggctccc agagagccat ggcgaccagg gtgtccccag 14280
   ttgtttctta aggtgcctct gaggggatct atttagttga aaagactgga ttcattcatt 14340
   caaccacagc aagttatctg ctaggtttga gctgtgccac ccccagtgta gcttcatgac 14400
   ttttctctgc tttttattcc aggtgagggc ttccacaact accaccacac ctttccctat 14460
   gactattctg ccagcgagta ccgctggcac atcaacttaa ccacgttctt catcgactgc 14520
   atggctgccc tgggtctggc ttatgaccgg aagaaagtat ccaaggctgc catcttggcc 14580
   aggattaaaa gaactggaga tgagagctac aagagtggct gagtttgggg tcccttgggt 14640
   tccttttctg aaagccagct gggcagaggt ttaatgttct gtttattaac tactgaataa 14700
   tgctaccagg atgctaaaga tgatgatgtt aacccgttcc agtacagtat tcttttaaaa 14760
   tttcttttaa gattgaaagc ctacaattct gcctttatga tgctaagctc atattcttgt 14820
   ttcttctctc tcttctagtc ccattgtcct tctcttggct ttgttcctgt caccttcctc 14880
   tctcttcccc ctcactgtgc cccaggcaag gagctggtca gtcgttggtg ggtttccagc 14940
   ttccaaagcc tagacctttc agtagtccaa aactggtgag cggtctttgc cccagatagc 15000
   tctttctttg agttgtcctg agctttaagg tgggtggctc aagggagaga ggtgataaaa 15060
   tcttctggga aagcccctgt tcattatttt cagcccagac ttttgctcaa tgacaaaaat 15120
   aactctattt tggcacaaag cttcgaaagc aggtaacttg tcaggggagg gagttagcat 15180
   gctgtgtgtg gtggggttga taaagaaggg ggaggtgagg tgggaaacca ggcaggaggc 15240
   tcctgctgtg atgggacact cggctgactg cccagtgagg gctttgcgcc ctggcacaca 15300
   gcatgcttcc tttctctcct gactctgggg aatggccgtg gaacttggca atgctagaac 15360
   tcaaaagcac atcccagtgt cccaatgtac gttaggctga ggataaagaa gcagcattta 15420
   gtttgtggca gcagtggtct ctgctgggga agaagtcttt cttgtctctt taataacagg 15480
   aagatttctt attccataga gtgagaaatc ttgaggttct ttccggaatt gctgaatcgg 15540
   caactcatgg aattgtcctc actcttttca tcttcccgct ctgccatctt tgggatacag 15600
   ctcccctcat agtaacaata aggtggctgc cgcattttga gacatcggaa ctgaggtgtg 15660
   ttggtggtac cgtggtgagc ttaactatct tcccaaaaag aaaggatttt agcaggtgga 15720
   ggtgggtccc acataaagat agttaaacct cggtactttg cttggaatac caacataatt 15780
   ctcttggact atttccctct gaaagagaag gaggcttgag aagaggaaga atgggggtgg 15840
   atggtctcct ttcctctctc tgctgaacag gagatggagg ggttgagggg cagggtctag 15900
   aggcagctcc tgagacataa cattgcaaac gaagggctct gggggattca gaaggttact 15960
   gagtaagtta ctggacgtcc tcctatggga agctggtcac acaggcaagt tagatattgg 16020
   ggttcatttc attcattcca gttgctgctt ggaataagga actagaaggc tgctccccac 16080
   agtgtgaaag cctttcactc ctgccttcta gcctcaagcg tactaccctg actatggatt 16140
   cctgctctgc cctgtctatc cgttttttcc tgccggttct atctcctccc tgaggttttt 16200
   cctttctctc tggagggcag gcctcctttg ggagtatgca aaggcagtga tggctgctgc 16260
   tctacaggca gcttcctctc ccacagtcag aatgctcagg gtcactgaac cactgtttct 16320
   cttcacaaag gtgagctagc tgccacctcc acgtggcctc cagagtctcc acctgcaccc 16380
   ttgtgctccc ctgccactcc aatgattcaa gacaaggcgg gcaaaccctc ctagaaacat 16440
   cccgggcacg ggcatccttt ctcataaggc acagccaagc caaatgctca cgttgtgcca 16500
   gtgagtcagc cacagagcaa aagagggttt gtggttagtc tcctctatct gggtcagaac 16560
   cagagagcag gctggatgcc ccctgcttgc tcggtaagcc tgcccagcct gagtcaatgc 16620
   tcacggctga cagtgcaatg cttgcagaaa caggagggag cctggtcttc actgggaagc 16680
   acaagaggcc aagacaagtt ccaaagtgcc tcactcgaaa gggaaccctg tcccctggag 16740
   ctagggtgta ccacaaagct ttggctgagt cttgggctga acagcgtctc tgttcagcaa 16800
   actaaccagc attccctaca gcacagccca aggcagacaa gagaatagaa gagggctgga 16860
   aaacaaaaaa taagaacctt ggcccactcc tgtccctgta acctcagtcg tcaacacaga 16920
   agcctggctt tactctaaag attggaaata tacaatacca gatgctctgt ccactgttga 16980
   gccccaggag tggaagggca gagagcattt cttcctgtat taactgagta aatggaggat 17040
   aaaggggttg ggctggacta gaggcatctt tgtcttttga gccattcttc tcagtagaaa 17100
   aaaaagctga tggaagatca ctgtagttca cattcccaga cccaagcacc tactctttgg 17160
   aaatgactgt tgggttagtt ttaattccac aggtcgtcag atgcctgctt tatagctgat 17220
   gatcaaaacc aacttttatc tttctattct aattgttttc aatggatctg atccatacca 17280
   taaccctaca caaggctgga tggggttctt aggccaaggg ccccagtgta tgtgtggatg 17340
   tgtagggtgg gagggcgggg agtaaggaat acttttttca aggttctaaa gctgaattca 17400
   aatgacgcat taatgaccca gaaactcaga tctgatagaa tctgaatttc taacaggcct 17460
   tgctttgtag gtgtactgac aacttatctg ggggccttac atctttttta atcggtgtta 17520
   cgtccgagcc tgctctgctc cctcactccc tctgcactcc ctctgtggcg ttcccttgcc 17580
   cctgagagcc tgcagaagtg gctggtagaa gtggggggct ggctggagaa ttatcagtat 17640
   gcaggattcc tttctgggct ttgttttgga aactttcctt agggctgttt ttattaagtg 17700
   cccacatttg acggaaggtg gaaggaattt gaatgtattt gatttataat gttattatta 17760
   ttattttttt agattaaaag atggttgtag catttaaaac ggaaaccttt tctcctggtt 17820
   agctagtatc ctgagtgtat tctctgtaag tgtagctcaa atgggtcagc gtgaagaaaa 17880
   gttaaagaaa gcacgatgtc aaggttacac gggtggttaa ggccagggcc tctcctacca 17940
   ctgtgccact gacttgctat gtgaccctgg gcaagtcatt taactataat gtgcctcagt 18000
   tttccttctg ttaaaatggg ataataatac tgacctacct caaagggcag ttttgaggcg 18060
   tgactaatgc tttttataaa gcattttggg atccttcagc agaggaattc tcttaagtcc 18120
   tgagtatttt tataataaca gtatccacca tgaactgtgt ccaccatgaa ccctgtgtcc 18180
   tggatgctgt cattaatctt tatggttctc tctgagaaat tgaataaacc caatagataa 18240
   gtggtggata actagtcaga cagaatctga gaatgcataa actcattgcc atggaaacat 18300
   acacaggata ccttttcctt gattgggtgg gattttttcc cctttttatg tgggatagta 18360
   gttatttgtg gcctaagaat aattttggaa taatttctat taatatcaac tctgaagcta 18420
   gttgtactga tctgagattg tgtttgttca taataaaagg gaagtgaatc tgattgcctt 18480
   gtgtctgaga ggttttttgc ctgtgagtca gtctcttggg acttaatctg ccaccctatg 18540
   tcctgttcct gtacctttaa gagagtaaaa aaagtagaag ctacaatggt tgattcacac 18600
   tctagtaacc cgccctgccc tccctgccct tcttaggggt aactttaggt taaactcagc 18660
   cttggcagca ggaagccagg agtctgctgc cattagatca gacatgttaa ccccagctgg 18720
   ctctttggcc ctggggaaat gccaggtctc tttcggtaac atgggccaaa atgatactga 18780
   gagaggaaag gagcagcagg tgacacttcc ctggaaaggg gttctgtacc tggcaaccca 18840
   ggctgggcca tcctgcttca ttcctctgaa atgtagcccc ttctgggaat aaagtggctt 18900
   cctgaaaatg ctgattaaca acagcaggaa gaaaggaaaa gcaatcctag agaagccttg 18960
   taaattcctg gaatagagag ggcttcatga tcatcaacta gaaaggcttt ggcttttact 19020
   gctttagcta agaatctttt tttttttttt tttttttttt gtctttttgc tatttctttg 19080
   ggtcgctcct gcagcatatg gaggttccca ggctaggggt ccacagcaac gcgggatccg 19140
   agccgcgtct gcaacctaca ccacagctca cagcaacacc agatccttaa cccactgagc 19200
   aggggcaggg accgaacccc caacctcatg attcctagtc ggattcgttg accactgcgc 19260
   cacgacggga actcagctaa gaatcttgat gtgcaaacga gaagggcttt ttttgaagag 19320
   gcttcatttt ccaacaggag ccacctaagc ttctgagcat tccccagacc ccccccttac 19380
   actttctagt gtaacgtcgc cctcaccttg gacaagcacg actctcagtg attcatccca 19440
   aatcaagtct atattcagtg aggccttgcc aggccttaat aacgtaaatt gatggtggca 19500
   ctttcttaat atctggcttt ctaccccttt ccctctattg tgtttaaaat gttcgggttg 19560
   gaccattttt ccattttata taatggcatg gttccgccca agtctctgtt tttggaggca 19620
   aagacatcag ccacatgaga aagggcagct gtgataaact atagtgagtc taggatgata 19680
   ctcatgaaac caaattcctt ggaacaatct atatcagata gagggattgg gaccatgcgt 19740
   caacagaaat ctcacaccct cttttttttt cttttctttt ttggtcgcac atgcagcaca 19800
   ttgaagttcc tatgtgacct atgccacagc tagggcaatg ttggatcctt aacccactgt 19860
   gccacagcag gaactcgcta tgacaacatt tggtgcagga aacccaaatt gtgagtccac 19920
   cagactcaat aacatttact ataaacagcc attgtagtga gtgcttgaca gtgcattatc 19980
   acagcatttc caaagcaggt gccatcatta ccctaatttt attgatgaaa ttccctagaa 20040
   gagaaagaac acaaggcgag ggagtatgca aggatgctgt gattcagctc agaactttgc 20100
   ttatttactg ctctccaaat gggtgtaaag tagataagca acttgataag tctggatgca 20160
   taagtcccat atatgtaagt agatgcctgg cgatattcag ttatgctcag aggtaaaact 20220
   tggtagcaat ggtacagagc ttagtttatg caaagtcaaa tcgcagatgt gtcctctgtt 20280
   ccagaggaac ccaactcact ccacctcccg acatgtttta acattcaacc acacctgagt 20340
   tttaaagctc tcagaccata gaaaccattt cttgggccgc tcccacggca tatggaggtt 20400
   cccaggctag gggtcgaatc ggagctgtag ccaccagcct acgccacagc cacagcaatg 20460
   tgggatccga gcctcatctg caacctacac cacagctcac ggcaatgctg gatccttaac 20520
   ccactgagca aggccaggga tcgaacccgc aaactcatgg ttcctagtca gatttgttaa 20580
   ccactgagcc atgatgggaa ctcctcctct atcgttttgc aaaccatact ccatccaccc 20640
   tgctcttgtg ttttgagatc tttaagggca tctcttcttt tagaaatgtc tcctgcccct 20700
   aatggatcag tgcttaataa aagtattgaa agacctcgtt tctagtttta aattctgtct 20760
   ctgtgacaaa ctgtgtgaaa tgtcacaggg agtgggaatt gcattctgtg acctgtagtt 20820
   aacttactaa actgcaggca aaaagccgat tcctgcatgc tgactttggg gcaaatacca 20880
   caagctctaa ttgcaggttc caagaagttg agattctata ctgggattgc ccctcccccg 20940
   cccggggcct gcccagcctg ctgcaaccca ggccacagag agctc 20985
<210> 2
   <211> 20
   <212> DNA
   <213> Artificial Sequence
<220>
   <223> Promoter-F
<400> 2
   acttccctag tgcccatcct 20
<210> 3
   <211> 20
   <212> DNA
   <213> Artificial Sequence
<220>
   <223> Promoter-R
<400> 3
   gatcactttc ccagggatga 20
<210> 4
   <211> 21
   <212> DNA
   <213> Artificial Sequence
<220>
   <223> 3UTR_F1
<400> 4
   aagtatccaa gggctgccat c 21
<210> 5
   <211> 20
   <212> DNA
   <213> Artificial Sequence
<220>
   <223> 3UTR_R1
<400> 5
   caattccgga aagaacctca 20
<210> 6
   <211> 21
   <212> DNA
   <213> Artificial Sequence
<220>
   <223> 3UTR_F2
<400> 6
   tggggaagaa gtctttcttg t 21
<210> 7
   <211> 20
   <212> DNA
   <213> Artificial Sequence
<220>
   <223> 3UTR_R2
<400> 7
   ggttcagtga ccctgagcat 20
<210> 8
   <211> 20
   <212> DNA
   <213> Artificial Sequence
<220>
   <223> 3UTR_F3
<400> 8
   tttcctgccg gttctatctc 20
<210> 9
   <211> 20
   <212> DNA
   <213> Artificial Sequence
<220>
   <223> 3UTR_R3
<400> 9
   gagtaggtgc ttgggtctgg 20
<210> 10
   <211> 20
   <212> DNA
   <213> Artificial Sequence
<220>
   <223> 3UTR_F4
<400> 10
   atggaggata aaggggttgg 20
<210> 11
   <211> 20
   <212> DNA
   <213> Artificial Sequence
<220>
   <223> 3UTR_R4
<400> 11
   acttgcccag ggtcacatag 20
<210> 12
   <211> 20
   <212> DNA
   <213> Artificial Sequence
<220>
   <223> 3UTR_F5
<400> 12
   gtcaaggtta cacgggtggt 20
<210> 13
   <211> 20
   <212> DNA
   <213> Artificial Sequence
<220>
   <223> 3UTR_R5
<400> 13
   caggacatag ggtggcagat 20
<210> 14
   <211> 24
   <212> DNA
   <213> Artificial Sequence
<220>
   <223> fordward primer for genotyping SNP3
<400> 14
   tgccagctct agcctttaaa tacc 24
<210> 15
   <211> 20
   <212> DNA
   <213> Artificial Sequence
<220>
   <223> reverse primer for genotyping SNP3
<400> 15
   cacgttgggt cggtgtgtct 20
<210> 16
   <211> 14
   <212> DNA
   <213> Artificial Sequence
<220>
   <223> G allele probe
<400> 16
   acccgcgcac agca 14
<210> 17
   <211> 16
   <212> DNA
   <213> Artificial Sequence
<220>
   <223> A allele probe
<400> 17
   agacccacgc acagca 16